# EUROPEAN PATENT APPLICATION

(11) **EP 4 785 927 A2**
(43) Date of publication of application: **05.08.2026**
(21) Application number: 26170924.0
(22) Date of filing: 01.11.2022
(51) Int. Cl.: A61K 9/00

(54) **PHARMACEUTICAL COMPOSITIONS OF HUMANIZED ANTI-CD40 ANTIBODIES**

(30) Priority: 05.11.2021 US 202163276363 P
(62) Divisional of application: 22890984.2
(71) Applicant: Kiniksa Pharmaceuticals, GmbH, 6302 Zug (CH)
(72) Inventor: KRANZ, James, Lexington, MA 02421 (US)
(74) Representative: Simmons & Simmons LLP (Munich)

(57) **Abstract**

The present disclosure relates to pharmaceutical compositions of anti-CD40 antibodies, such as humanized anti-CD40 antibodies. The pharmaceutical compositions may be used in various therapeutic, prophylactic and diagnostic methods. The antibodies block the ability of CD40 to bind CD154 without activating the cell expressing CD40 (e.g., a B cell).

## Description

### Sequence Listing

This application contains a Sequence Listing which has been filed electronically in Extensible Markup Language (XML) format and is hereby incorporated by reference in its entirety. Said XML copy, created on 31 March 2026, is named P11376EP01.XML and is 20.5 KB in size.

### Background

Suppression of the immune system, particularly the humoral immune system, is beneficial in organ transplantation and treatment of autoimmune disorders.

One target for suppressing the immune system is the CD40/CD154 interaction. CD40 is expressed primarily on the surface of B lymphocytes and other antigen-presenting cells (APCs) such as dendritic cells and macrophages. CD154 is expressed primarily on the surface of T cells. The interaction between these two proteins is associated with B cell activation, which triggers cytokine expression as well as expression of cell surface markers including CD23, CD80, and CD86. Antibodies (e.g., humanized antibodies) that target the CD40/CD154 interaction have been developed. However, formulating these antibodies or antigen-binding fragments thereof into suitable pharmaceutical compositions for administration in vivo is necessary for effective treatment.

### Summary

In general, featured is a pharmaceutical composition containing a humanized anti-CD40 antibody or antigen-binding fragment thereof with a heavy chain variable region having an amino acid sequence with at least 80% (e.g., at least 85%, 90%, 95%, 97%, 99%,or 100%) sequence identity to the amino acid sequence set forth in SEQ ID NO: 9, and a light chain variable region having an amino acid sequence with at least 80% (e.g., at least 85%, 90%, 95%, 97%, 99%,or 100%) sequence identity to the amino acid sequence set forth in SEQ ID NO: 10. In one embodiment, the humanized anti-CD40 antibody is KPL-404. The pharmaceutical composition includes polysorbate 20, acetate, and one or more polar excipients. The polar excipient may be or may include, for example, a sugar, a polyol, or an amino acid. In some embodiments, the sugar is, for example, sucrose, trehalose, fructose, lactose, dextrose, or mannitol. In some embodiments, the polyol is, for example, polyethylene glycol or sorbitol. In some embodiments, the amino acid is one or more of alanine, arginine, aspartic acid, asparagine, carnitine, citrulline, ornithine, glycine, glutamic acid, glutamine, glycine, isoleucine, leucine, lysine, methionine, phenylalanine, proline, serine, threonine, tyrosine, and valine. In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 45 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) of the polar excipient. In some embodiments, the pharmaceutical composition includes about 50 mM of the polar excipient. In some embodiments, the pharmaceutical composition includes about 100 mM of the polar excipient. In some embodiments, the pharmaceutical composition includes about 150 mM of the polar excipient. In some embodiments, the pharmaceutical composition includes about 200 mM of the polar excipient.

In some embodiments, the pharmaceutical composition includes from about 1% (w/v) to about 10% (w/v) (e.g., about 2% (w/v) to about 8% (w/v), e.g., about 1% (w/v), 1.5 % (w/v), 2% (w/v), 2.5% (w/v), 3% (w/v), 3.5% (w/v), 4% (w/v), 4.5% (w/v), 5% (w/v), 5.5% (w/v), 6% (w/v), 6.5% (w/v), 7% (w/v), 7.5% (w/v), 8% (w/v), 8.5% (w/v), 9% (w/v), 9.5% (w/v), 10% (w/v)) of the polar excipient. In some embodiments, the pharmaceutical composition includes about 2.5% (w/v) of the polar excipient. In some embodiments, the pharmaceutical composition includes about 7% (w/v) of the polar excipient.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

The composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

In a first aspect, featured is a pharmaceutical composition containing a humanized anti-CD40 antibody or antigen-binding fragment thereof with a heavy chain variable region having an amino acid sequence with at least 80% (e.g., at least 85%, 90%, 95%, 97%, 99%,or 100%) sequence identity to the amino acid sequence set forth in SEQ ID NO: 9, and a light chain variable region having an amino acid sequence with at least 80% (e.g., at least 85%, 90%, 95%, 97%, 99%,or 100%) sequence identity to the amino acid sequence set forth in SEQ ID NO: 10. In an embodiment, the humanized anti-CD40 antibody is KPL-404. The pharmaceutical composition includes polysorbate 20, acetate, and a polar excipient. For example, the polar excipient may include arginine and glutamate.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 45 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) arginine. In some embodiments, the pharmaceutical composition includes about 50 mM arginine. In some embodiments, the pharmaceutical composition includes about 100 mM arginine.

In some embodiments, the pharmaceutical composition includes from about 1 mM to about 200 mM (e.g., about 50 mM to about 150 mM, e.g., about 50 mM to about 100 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) glutamate. In some embodiments, the pharmaceutical composition includes about 50 mM glutamate. In some embodiments, the pharmaceutical composition includes about 100 mM glutamate.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM acetate; from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) arginine; from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) glutamate; and from about 0.001% to about 0.1% (e.g., 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01%) polysorbate 20. The composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

In some embodiments, the pharmaceutical composition includes about 50 mM acetate, about 100 mM arginine, 100 mM glutamate, and about 0.01% polysorbate 20. In some embodiments, the pharmaceutical composition includes about 50 mM acetate, about 100 mM arginine, 100 mM glutamate, and about 0.02% polysorbate 20. In some embodiments, the pharmaceutical composition includes about 50 mM acetate, about 50 mM arginine, 50 mM glutamate, and about 0.01% polysorbate 20. In some embodiments, the pharmaceutical composition includes about 50 mM acetate, about 50 mM arginine, 50 mM glutamate, and about 0.02% polysorbate 20. In some embodiments, the pharmaceutical composition includes about 50 mM acetate, about 100 mM arginine, 50 mM glutamate, and about 0.01% polysorbate 20. In some embodiments, the pharmaceutical composition includes about 50 mM acetate, about 100 mM arginine, 50 mM glutamate, and about 0.02% polysorbate 20. The composition may have a pH of about 5.4.

In a second aspect, featured is a pharmaceutical composition including a humanized anti-CD40 antibody or antigen-binding fragment thereof having a heavy chain variable region having an amino acid sequence with at least 80% (e.g., at least 85%, 90%, 95%, 97%, 99%,or 100%) sequence identity to the amino acid sequence set forth in SEQ ID NO: 9, and a light chain variable region having an amino acid sequence with at least 80% (e.g., at least 85%, 90%, 95%, 97%, 99%,or 100%) sequence identity to the amino acid sequence set forth in SEQ ID NO: 10. The pharmaceutical composition includes polysorbate 20, acetate, and a polar excipient. The polar excipient may be or may include sucrose.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 1% (w/v) to about 10% (w/v), e.g., 1%, 2%, 3%, 4%, 5%, 6%, 7%, 8%, 9%, or 10% (w/v), sucrose. In some embodiments, the pharmaceutical composition includes about 7% (w/v) sucrose.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 1% (w/v) to about 10% (w/v), e.g., 1%, 2%, 3%, 4%, 5%, 6%, 7%, 8%, 9%, or 10% (w/v), e.g., about 7%, sucrose, and from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20. The composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

In some embodiments, the pharmaceutical composition includes about 50 mM acetate, about 7% (w/v) sucrose, and about 0.01% polysorbate 20. The composition may have a pH of about 5.4.

In some embodiments, the pharmaceutical composition includes about 50 mM acetate, about 7% (w/v) sucrose, and about 0.02% polysorbate 20. The composition may have a pH of about 5.4.

In a third aspect, featured is a pharmaceutical composition including a humanized anti-CD40 antibody or antigen-binding fragment thereof having a heavy chain variable region having an amino acid sequence with at least 80% (e.g., at least 85%, 90%, 95%, 97%, 99%,or 100%) sequence identity to the amino acid sequence set forth in SEQ ID NO: 9, and a light chain variable region having an amino acid sequence with at least 80% (e.g., at least 85%, 90%, 95%, 97%, 99%,or 100%) sequence identity to the amino acid sequence set forth in SEQ ID NO: 10. The pharmaceutical composition includes polysorbate 20, acetate, and a polar excipient. The polar excipient may be or may include sorbitol. The pharamcetuical composition may further include sodium chloride.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 0.1% (w/v) sorbitol to about 5% (w/v), e.g., about 1mM to about 5mM, e.g., about 2mM to about 3mM, e.g., 0.2%, 0.3%, 0.4%, 0.5%, 0.6%, 0.7%, 0.8%, 0.9%, 1%, 2%, 3%, 4%, or 5% (w/v), sorbitol. In some embodiments, the pharmaceutical composition includes about 2.5% (w/v) sorbitol.

In some embodiments, the pharmaceutical composition includes from about 1 mM to about 100 mM (e.g., about 10 mM to about 90 mM, e.g., about 50 mM to about 90 mM, e.g., about 60 mM to about 80 mM, e.g., about 65 mM to about 75 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, or 100 mM) sodium chloride. In some embodiments, the pharmaceutical composition includes about 70 mM sodium chloride.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

In some embodiments, the pharmaceutical composition includes from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 0.1% (w/v) sorbitol to about 5% (w/v), e.g., 0.2%, 0.3%, 0.4%, 0.5%, 0.6%, 0.7%, 0.8%, 0.9%, 1%, 2%, 3%, 4%, or 5% (w/v), e.g., about 2.5%, sorbitol, from about 1 mM to about 100 mM (e.g., about 10 mM to about 90 mM, e.g., about 50 mM to about 90 mM, e.g., about 60 mM to about 80 mM, e.g., about 65 mM to about 75 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, or 100 mM, e.g., about 70 mM) sodium chloride, and from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20. The composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

In some embodiments, the pharmaceutical composition includes about 50 mM acetate, about 2.5% (w/v) sorbitol, about 70 mM NaCl, and about 0.01% polysorbate 20. In some embodiments, the pharmaceutical composition includes about 50 mM acetate, about 2.5% (w/v) sorbitol, about 70 mM NaCl, and about 0.02% polysorbate 20. The composition may have pH of about 5.4.

In some embodiments, the pharmaceutical composition of any one of the first, second, and third aspects has a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

In some embodiments of any one of the first, second, and third aspects, the anti-CD40 antibody or antigen-binding fragment thereof is at a concentration of from about 0.01 mg/mL to about 300 mg/mL, e.g., about 100 mg/mL to about 250 mg/mL, about 150 mg/mL to about 250 mg/mL, or about 100 mg/mL to about 200 mg/mL (e.g., 0.02 mg/mL, 0.03 mg/mL, 0.04 mg/mL, 0.05 mg/mL, 0.06 mg/mL, 0.07 mg/mL, 0.08 mg/mL, 0.09 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL, 2 mg/mL, 3 mg/mL, 4 mg/mL, 5 mg/mL, 6 mg/mL, 7 mg/mL, 8 mg/mL, 9 mg/mL, or 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL, 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL). In some embodiments, the anti-CD40 antibody or antigen-binding fragment thereof is at a concentration of about 200 mg/mL.

In some embodiments of any one of the first, second, and third aspects, the composition is formulated in a volume of about 0.1 mL to about 2.0 mL (e.g., about 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, or 2.0 mL, 2.5 mL, 3.0 mL, 3.5 mL, 4.0 mL 4.5 mL, 5.5 mL or 5.5 mL). In some embodiments, the composition is formulated in a volume of about 2.0 mL.

In some embodiments of any one of the first, second, and third aspects, the composition is formulated in a volume of about 0.1 mL to about 2.0 mL (e.g., about 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, or 2.0 mL, 2.5 mL, 3.0 mL, 3.5 mL, 4.0 mL 4.5 mL, 5.5 mL or 5.5 mL, e.g., about 2.0 mL, and the antibody or antigen-binding fragment thereof is at a concentration of from about 50 mg/mL to about 300 mg/mL (e.g., 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL, 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL, e.g., about 200 mg/mL.

In some embodiments, at least 95% (e.g., at least 98%) of the humanized anti-CD40 antibody or antigen-binding fragment thereof in the composition is in a monomeric form. In some embodiments, less than 5% (e.g., less than 2%) of the humanized anti-CD40 antibody or antigen-binding fragment is present in the composition as a high molecular weight (HMW) species (e.g., in an aggregated form).

In some embodiments, an amount of monomeric species of the humanized anti-CD40 antibody or antigen-binding fragment in the composition decreases to no less than 95% (e.g., no less than 98%) following storage at 2-8 °C for 12 months. In some embodiments, an amount of HMW species of the humanized anti-CD40 antibody or antigen-binding fragment in the composition increases to no more than 5% (e.g., no more than 2%) following storage at 2-8 ° C for 12 months. In a fourth aspect, featured is a kit that includes a pharmaceutical composition of any one of the first second, and third aspects, as described herein, and instructions for use thereof.

In a fifth aspect, featured is the use of a pharmaceutical composition of any one of the first second, and third aspects, as described herein, in the manufacture of a medicament for suppressing the immune system in a human subject.

In a sixth aspect, featured is a pharmaceutical composition of any one of the first second, and third aspects, as described herein, for use in suppressing the immune system in a human subject.

In a seventh aspect, the invention features a method of suppressing the immune system in a human subject by administering a pharmaceutical composition of any one of the first second, and third aspects, as described herein, to a subject (e.g., a subject in need thereof, such as a human subject).

### Brief Description of the Drawings

The patent or application file contains at least one drawing executed in color. Copies of this patent or patent application publication with color drawing(s) will be provided by the Office upon request and payment of the necessary fee.
**FIG.** 1 is a set of graphs showing SEC results presented graphically for %Main, %HMW and %LMW species of KPL-404 for Formulations A, 1, and 2.
**FIGS. 2A** **and** **2B** are graphs showing comparison of particle concentrations from biophysical characterization studies. **FIG. 2A** shows 200 mg/mL KPL-404 Formulations A-C (no PS20 in Formulation C), and **FIG. 2B** shows high concentrations of KPL-404 in Formulations A-C.
**FIG. 3** is a graph showing viscosity vs. shear rate for high concentration KPL-404 formulations.
**FIG. 4** is a graph showing Total KPL-404 species Population by CG-MALS for formulation A.
**FIG. 5** is a graph showing Total KPL-404 species Population by CG-MALS for formulation B.
**FIG. 6** is a graph showing Total KPL-404 species Population by CG-MALS for formulation C.
**FIG. 7** is a graph showing Exponential depe ndence of measured viscosity vs protein concentration for formulation A (circles), B (squares), and C (triangles).
**FIG. 8** is a graph showing Instron Force Profiles, KPL-404 in Formulation A, with Injection times from a Standard Ypsomate Autoinjector (2.25 mL).
**FIG. 9** is a graph showing Instron Force Profiles, KPL-404 in Formulation B, with Injection times from a Standard Ypsomate Autoinjector (2.25 mL).
**FIGS. 10A** **and** **10B** are graphs showing Instron Force Profiles, KPL-404 in Formulation C, with Injection times from a Standard Ypsomate **(****FIG. 10A****)** or Ypsomate Pro **(****FIG. 10B****)** Autoinjector (2.25 mL).
**FIG. 11** is a set of SEC Chromatograms of Formulations A, B, and C, t=0 (initial). The right side shows an enhanced view of the left panel.
**FIG. 12** is a set of SEC Chromatograms of Formulations A, B, and C, t=2w, 40°C. The right side shows an enhanced view of the left panel.
**FIG. 13** is a graph showing SEC %Main as a function of time, 5°C storage.
**FIG. 14** is a graph showing SEC %Main as a function of time, 25°C storage.
**FIG. 15** is a graph showing SEC %Main as a function of time, 40°C storage.

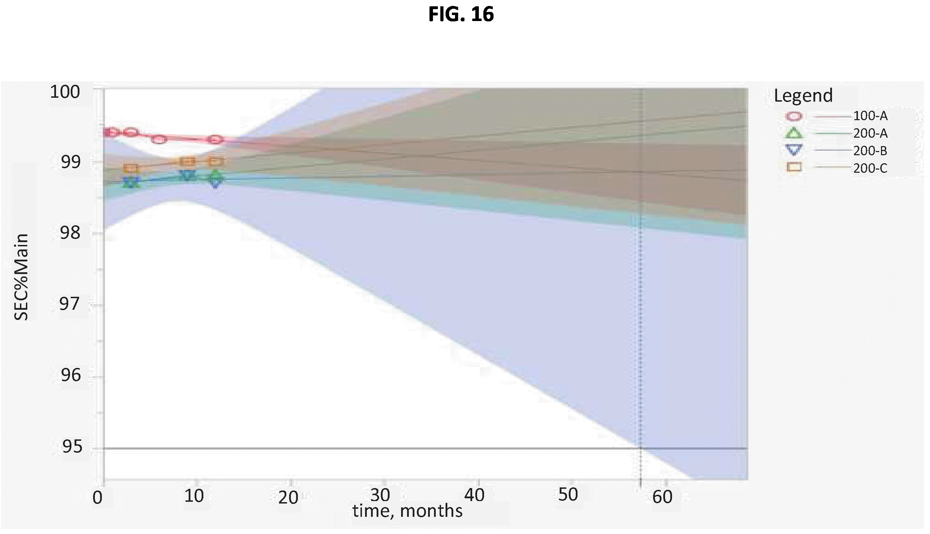

**FIG. 16** is a graph showing SEC %Main as a function of time, -70°C storage.

### Detailed Description

The present disclosure relates to pharmaceutical compositions containing anti-CD40 antibodies and antigen-binding fragments of the antibody that may be used in various therapeutic, prophylactic, diagnostic, and other methods. The pharmaceutical compositions contain antibodies or antigen-binding fragments thereof that can block the ability of CD40 to bind CD154 and do so without activating the cell expressing CD40 (e.g., a B cell). The pharmaceutical compositions described herein may be used to treat autoimmune diseases and disorders. In general, the antibody or antigen-binding fragment thereof is derived from the murine 2C10 antibody and humanized variants thereof, e.g., as described in PCT Pub. Nos. WO 2012/125569 and WO 2017/040932, which are herein incorporated by reference in their entirety, including the sequences described therein. The pharmaceutical compositions contain combinations of specific carriers and excipients that impart surprisingly beneficial properties to the compositions, including the ability to formulate the drug (the anti-CD40 antibodies and antigen-binding fragments thereof) at a high concentration (as described herein), to enhance stability of the composition (e.g., extended shelf-life), to reduce aggregation of the drug, and to improve viscosity parameters of the composition. The components of the pharmaceutical composition are described in more detail below.

### Antibodies and Antigen-Binding Fragments Thereof

The pharmaceutical compositions described herein include an antibody or antigen-binding fragment thereof, e.g., derived from the murine 2C10 antibody, including, without limitation, the humanized anti-CD40 antibody identified as KPL-404. The heavy chain variable regions, light chain variable regions, and CDRs of certain anti-CD40 antibodies described herein are shown in **Table 1.**

**Table 1: 2C10 and KPL-404 antibody sequences**

| **Name** | **Chain, Region** | **Sequence** | **SEQ ID NO:** |
|---|---|---|---|
| 2C10 (Murine antibody) | Heavy chain, variable region | QVQLQQSGAELAKPGASVKMSCKASGYTFTNYWMH WVKQRPGQGLEWIGYINPSNDYTKYNQKFKDKATLTA DKSSNTAYMQLGSLTSEDSAVYYCARQGFPYWGQG TLVTVSA | 1 |
| 2C10 (Murine antibody) | Light chain, variable region | QIVLTQSPAIMSASPGEKVTMTCSASSSVSYMHWYHQ RSGTSPKRWIYDTSKLASGVPARFSGSGSGTSYSLTI SSMEAEDAATYYCHQLSSDPFTFGSGTKLEIK | 2 |
| 2C10 and KPL-404 | Heavy chain, CDR1 | YTFTNYWMH | 3 |
| 2C10 and KPL-404 | Heavy chain, CDR2 | YINPSNDYTKYNQKFKD | 4 |
| 2C10 and KPL-404 | Heavy chain, CDR3 | QGFPY | 5 |
| 2C10 and KPL-404 | Light chain, CDR1 | SASSSVSYMH | 6 |
| 2C10 and KPL-404 | Light chain, CDR2 | DTSKLAS | 7 |
| 2C10 and KPL-404 | Light chain, CDR3 | HQLSSDPFT | 8 |
| KPL-404 | Heavy chain, variable region | QVQLVQSGAEVKKPGASVKVSCKASG YTFTNYWMH WVRQAPGQRLEWIG YINPSNDYTKYNQKFKD RATLTADKSANTAYM ELSSLRSEDTAVYYCAR QGFPY WGQGTLVTVSS | 9 |
| KPL-404 | Light chain, variable region | EIVLTQSPATLSLSPGERATLSC SASSSVSYMH WYQQKPGQAPRRWIY DTSKLAS GVPARFSGSGSGTDYTLTISSLEPEDFAVYYC HQLSSDPFT FGGGTKVEIK | 10 |
| KPL-404 | Heavy chain (IgG4 constant domain) | QVQLVQSGAEVKKPGASVKVSCKASGYTFTNYWMH WVRQAPGQRLEWIGYINPSNDYTKYNQKFKDRATLTA DKSANTAYMELSSLRSEDTAVYYCARQGFPYWGQGT LVTVSSASTKGPSVFPLAPCSRSTSESTAALGCLVKD YFPEPVTVSWNSGALTSGVHTFPAVLQSSGLYSLSSV VTVPSSSLGTKTYTCNVDHKPSNTKVDKRVESKYGPP CPPCPAPEFLGGPSVFLFPPKPKDTLMISRTPEVTCVV VDVSQEDPEVQFNWYVDGVEVHNAKTKPREEQFNST YRVVSVLTVLHQDWLNGKEYKCKVSNKGLPSSIEKTIS KAKGQPREPQVYTLPPSQEEMTKNQVSLTCLVKGFY PSDIAVEWESNGQPENNYKTTPPVLDSDGSFFLYSRL TVDKSRWQEGNVFSCSVMHEALHNHYTQKSLSLSPG K | 11 |
| KPL-404 | Light chain (kappa constant domain) | EIVLTQSPATLSLSPGERATLSCSASSSVSYMHWYQQ KPGQAPRRWIYDTSKLASGVPARFSGSGSGTDYTLTI SSLEPEDFAVYYCHQLSSDPFTFGGGTKVEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC | 12 |
| 2C10_h1 | Heavy chain, variable region | QVQLVQSGAEVKKPGASVKVSCKASGYTFTNYWMH WVRQAPGQRLEWMGYINPSNDYTKYNQKFKDRVTIT RDTSASTAYMELSSLRSEDTAVYYCAR QGFPYWGQGTLVTVSS | 13 |
| 2C10_h2 | Heavy chain, variable region | QVQLVQSGAEVKKPGASVKVSCKASGYTFTNYWMH WVRQAPGQRLEWMGYINPSNDYTKYNQKFKDRVTIT ADKSASTAYMELSSLRSEDTAVYYCARQGFPYWGQG TLVTVSS | 14 |
| 2C10_I1 | Light chain, variable region | EIVLTQSPATLSLSPGERATLSCSASSSVSYMHWYQQ KPGQAPRLLIYDTSKLASGIPARFSGSGSGTDFTLTISS LEPEDFAVYYCHQLSSDPFTFGGGTKVEIK | 15 |
| 2C10HP | Heavy chain, variable region | QVQLVQSGAEVKKPGASVKVSCKASGYTFTNYWMH WVRQAPGQRLEWIGYINPSNDYTKYNQKFKDRATLTA DKSANTAYMELSSLRSEDTAVYYCARQGFPYWGQGT LVTVSS | 16 |
| 2C10HB1 | Heavy chain, variable region | QVQLVQSGAEVKKPGASVKVSCKASGYTFTNYWMH WVRQAPGQRLEWIGYINPSNDYTKYNQKFKDRATLTA DTSTNTAYMELSSLRSEDTAVYYCARQGFPYWGQGT LVTVSS | 17 |
| 2C10HB2 | Heavy chain, variable region | QVQLVQSGAEVKKPGSSVKVSCKASGYTFTNYWMH WVRQAPGQGLEWIGYINPSNDYTKYNQKFKDKATITA DESTNTAYMELSSLRSEDTAVYYCARQGFPYWGQGT LVTVSS | 18 |
| 2C10KP | Light chain, variable region | EIVLTQSPATLSLSPGERATLSCSASSSVSYMHWYQQ KPGQAPRRWIYDTSKLASGVPARFSGSGSGTDYTLTI SSLEPEDFAVYYCHQLSSDPFTFGGGTKVEIK | 19 |
| 2C10KB1 | Light chain, variable region | DIQMTQSPSTLSASVGDRVTITCSASSSVSYMHWYQQ KPGKAPKLLIYDTSKLASGVPARFSGSGSGTEFTLTIS SLQPDDFATYYCHQLSSDPFTFGQGTKVEVK | 20 |
| 2C10KB2 | Light chain, variable region | EIVLTQSPATLSLSPGERATLSCSASSSVSYMHWYQQ KPGQAPRLLIYDTSKLASGIPARFSGSGSGTDFTLTISS LEPEDFAVYYCHQLSSDPFTFGQGTKLEIK | 21 |

In certain embodiments, the antibody or antigen-binding fragment thereof includes a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the heavy chain variable region amino acid sequence as set forth in SEQ ID NO: 9. In a certain embodiment, the antibody includes the heavy chain amino acid sequence as set forth in SEQ ID NO: 11.

In certain embodiments, the antibody or antigen-binding fragment thereof includes a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a light chain variable region amino acid sequence as set forth in SEQ ID NO: 10. In a certain embodiment, the antibody includes the light chain amino acid sequence as set forth in SEQ ID NO: 12.

In certain embodiments, the antibodies or antigen-binding fragments thereof each include both a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a heavy chain variable region amino acid sequence as set forth in SEQ ID NO: 9, and a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to a variable light chain amino acid sequence as set forth in SEQ ID NO: 10. In a certain embodiment, the antibody includes the heavy chain amino acid sequence as set forth in SEQ ID NO: 11 and the light chain amino acid sequence as set forth in SEQ ID NO: 12. In a certain embodiment, the anti-CD40 antibody is KPL-404.

In certain embodiments, the antibodies or antigen-binding fragments thereof used to produce a pharmaceutical composition described herein include a heavy chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to a heavy chain variable region amino acid sequence set forth in any one of SEQ ID NOs: 13, 14, 16, 17, or 18, and a light chain variable region including an amino acid sequence having at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% sequence identity to a variable light chain amino acid sequence set forth in any one of SEQ ID NOs: 15, 19, 20, or 21.

In certain embodiments, a heavy chain variable region of the antibody or antigen-binding fragment thereof includes complementarity determining regions (CDRs) that are at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the CDRs of a heavy chain variable region of the KPL-404 antibody (CDR1, CDR2 and CDR3 as set forth in SEQ ID NOs: 3, 4, 5, respectively).

In certain embodiments, the light chain variable region of the antibody or antigen-binding fragment thereof includes CDRs that are at least about 70%, at least about 75%, at least about 80%, at least about 85%, at least about 90%, at least about 95%, at least about 99%, about 70%, about 75%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% or about 100% identical to the CDRs of a light chain variable region of the KPL-404 antibody (CDR1, CDR2 and CDR3 as set forth in SEQ ID NOs: 6, 7, 8, respectively).

In certain embodiments, the heavy chain includes the CDRs as set forth in SEQ ID NOs: 3-5, respectively, and the light chain includes the CDRs as set forth in SEQ ID NOs: 6-8, respectively.

In certain embodiments, the heavy chain has at least 80% (e.g., at least 85%, 90%, 95%, 97%, 99%, or 100%) sequence identity to SEQ ID NO: 9 and the CDRs set forth in SEQ ID NOs: 3-5, respectively, and the light chain has at least 80% (e.g., at least 85%, 90%, 95%, 97%, 99%, or 100%) sequence identity to SEQ ID NO: 10 and the CDRs set forth in SEQ ID NOs: 6-8, respectively.

Also within the scope of the disclosure are antibodies or antigen-binding fragments thereof in which specific amino acids have been substituted, deleted, or added. These alternations do not have a substantial effect on the peptide's biological properties such as binding activity. For example, antibodies may have amino acid substitutions in the framework region, such as to improve binding to the antigen. In another example, a selected, small number of acceptor framework residues can be replaced by the corresponding donor amino acids. The donor framework can be a mature or germline human antibody framework sequence or a consensus sequence. Guidance concerning how to make phenotypically silent amino acid substitutions is provided in, e.g., Bowie et al. (Science, 247: 1306-1310, 1990), Cunningham et al. (Science, 244: 1081-1085, 1989), Ausubel (ed.) (Current Protocols in Molecular Biology, John Wiley and Sons, Inc., 1994), T. Maniatis, E. F. Fritsch and J. Sambrook (Molecular Cloning: A Laboratory Manual, Cold Spring Harbor laboratory, Cold Spring Harbor, N.Y., 1989), Pearson (Methods Mol. Biol. 243:307-31, 1994), and Gonnet et al. (Science 256:1443-45, 1992); each of which is incorporated herein by reference.

The peptides described herein may be a functionally active variant of the antibodies or antigen-binding fragments thereof disclosed herein, e.g., with less than about 30%, about 25%, about 20%, about 15%, about 10%, about 5% or about 1% amino acid residues substituted or deleted but that retain essentially the same immunological properties including, but not limited to, binding to CD40.

The antibodies or antigen-binding fragments thereof may also include variants, analogs, orthologs, homologs and derivatives of peptides, that exhibit a biological activity, e.g., binding of an antigen such as CD40. The peptides may contain one or more analogs of an amino acid (including, for example, non-naturally occurring amino acids, amino acids which only occur naturally in an unrelated biological system, modified amino acids from mammalian systems etc.), peptides with substituted linkages, as well as other modifications known in the art.

The antibody or antigen-binding fragment thereof can be derivatized or linked to another functional molecule. For example, an antibody can be functionally linked (by chemical coupling, genetic fusion, noncovalent interaction, etc.) to one or more other molecular entities, such as another antibody, a detectable agent, an immunosuppressant, a cytotoxic agent, a pharmaceutical agent, a protein or peptide that can mediate association with another molecule (such as a streptavidin core region or a polyhistidine tag), amino acid linkers, signal sequences, immunogenic carriers, or ligands useful in protein purification, such as glutathione-S-transferase, histidine tag, and staphylococcal protein A. Cytotoxic agents may include radioactive isotopes, chemotherapeutic agents, and toxins such as enzymatically active toxins of bacterial, fungal, plant, or animal origin, and fragments thereof. Such cytotoxic agents can be coupled to the humanized antibodies of the present disclosure using standard procedures, and used, for example, to treat a patient indicated for therapy with the antibody.

One type of derivatized protein is produced by crosslinking two or more proteins (of the same type or of different types). Suitable crosslinkers include those that are heterobifunctional, having two distinct reactive groups separated by an appropriate spacer (e.g., m-maleimidobenzoyl-N-hydroxysuccinimide ester) or homobifunctional (e.g., disuccinimidyl suberate). Useful detectable agents with which a protein can be derivatized (or labeled) include fluorescent agents, various enzymes, prosthetic groups, luminescent materials, bioluminescent materials, and radioactive materials. Non-limiting, exemplary fluorescent detectable agents include fluorescein, fluorescein isothiocyanate, rhodamine, and phycoerythrin. A protein or antibody can also be derivatized with detectable enzymes, such as alkaline phosphatase, horseradish peroxidase, beta-galactosidase, acetylcholinesterase, glucose oxidase and the like. A protein can also be derivatized with a prosthetic group (e.g., streptavidin/biotin and avidin/biotin).

In another embodiment, the humanized anti-CD40 antibody or its fragment is used unlabeled and detected with a labeled antibody that binds the humanized anti-CD40 antibody or its fragment.

### Pharmaceutical compositions

The present disclosure features pharmaceutical compositions containing an antibody or antigen-binding fragment thereof as described herein (e.g., an antibody or antigen-binding fragment thereof containing a heavy chain variable region having at least 80%, 85%, 90%, 95%, 97%, 99%, or 100% sequence identity to SEQ ID NO: 9 and a light chain variable region having at least 80%, 85%, 90%, 95%, 97%, 99%, or 100% sequence identity to SEQ ID NO: 10, formulated together with a pharmaceutically acceptable carrier). The present disclosure also features pharmaceutical compositions containing an antibody or antigen-binding fragment thereof as described herein (e.g., an antibody or antigen-binding fragment thereof containing a heavy chain having at least 80%, 85%, 90%, 95%, 97%, 99%, or 100% sequence identity to SEQ ID NO: 11 and a light chain having at least 80%, 85%, 90%, 95%, 97%, 99%, or 100% sequence identity to SEQ ID NO: 12, formulated together with a pharmaceutically acceptable carrier). The pharmaceutical composition containing the antibody or antigen-binding fragment thereof can be used, e.g., for the treatment of a disease or disorder, as described herein. The pharmaceutical compositions include polysorbate 20, acetate, and one or more polar excipients. The polar excipient may be or may include, for example, a sugar, a polyol, or an amino acid. In some embodiments, the sugar is, for example, sucrose, trehalose, fructose, lactose, dextrose, or mannitol. In some embodiments, the polyol is, for example, polyethylene glycol or sorbitol. In some embodiments, the amino acid is one or more of alanine, arginine, aspartic acid, asparagine, carnitine, citrulline, ornithine, glycine, glutamic acid, glutamine, glycine, isoleucine, leucine, lysine, methionine, phenylalanine, proline, serine, threonine, tyrosine, and valine. The acetate may include, for example, sodium acetate (e.g., in salt form) or acetate in its ionic form. In some embodiments, the polar excipient is, for example sucrose, arginine, glutamate, sorbitol, or a combination thereof. In some embodiments, the polar excipient is sucrose. In some embodiments the polar excipient is arginine. In some embodiments the polar excipient is glutamate. In some embodiments, the polar excipient is a mixture of arginine and glutamate. In some embodiments, the polar excipient is sorbitol.

In some embodiments, the pharmaceutical composition includes from about 5 mM to about 100 (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 45 mM, 50 mM, 60 mM, 70 mM, 80 mM, 90 mM, or 100 mM) acetate. In some embodiments, the pharmaceutical composition includes about 50 mM acetate.

In some embodiments, the pharmaceutical composition includes from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) of the polar excipient. In some embodiments, the pharmaceutical composition includes about 50 mM of the polar excipient. In some embodiments, the pharmaceutical composition includes about 100 mM of the polar excipient. In some embodiments, the pharmaceutical composition includes about 150 mM of the polar excipient. In some embodiments, the pharmaceutical composition includes about 200 mM of the polar excipient.

In some embodiments, the pharmaceutical composition includes from about 1% (w/v) to about 10% (w/v) (e.g., about 2% (w/v) to about 8% (w/v), e.g., about 1% (w/v), 1.5 % (w/v), 2% (w/v), 2.5% (w/v), 3% (w/v), 3.5% (w/v), 4% (w/v), 4.5% (w/v), 5% (w/v), 5.5% (w/v), 6% (w/v), 6.5% (w/v), 7% (w/v), 7.5% (w/v), 8% (w/v), 8.5% (w/v), 9% (w/v), 9.5% (w/v), 10% (w/v)) of the polar excipient. In some embodiments, the pharmaceutical composition includes about 2.5% (w/v) of the polar excipient. In some embodiments, the pharmaceutical composition includes about 7% (w/v) of the polar excipient.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20. In some embodiments, the pharmaceutical composition includes about 0.01% polysorbate 20. In some embodiments, the pharmaceutical composition includes about 0.02% polysorbate 20.

The composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0, e.g., about 5.4.

The pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) of the polar excipient, and from about 0.001% to about 0.1% (e.g., 0.001%, about 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20. The pharmaceutical composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

The pharmaceutical composition can include, e.g., an amount of an anti-CD40 antibody or antigen-binding fragment thereof described herein (e.g., KPL-404), such as, e.g., about 100 mg/mL to about 300 mg/mL, e.g., about 100 mg/mL to about 250 mg/mL, about 150 mg/mL to about 250 mg/mL, or about 100 mg/mL to about 200 mg/mL (e.g., about 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL and 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL).

In some embodiments, at least 95% (e.g., at least 98%) of the humanized anti-CD40 antibody or antigen-binding fragment thereof in the composition is in a monomeric form. In some embodiments, less than 5% (e.g., less than 2%) of the humanized anti-CD40 antibody or antigen-binding fragment is present in the composition as a high molecular weight (HMW) species (e.g., in an aggregated form).

In some embodiments, an amount of monomeric species of the humanized anti-CD40 antibody or antigen-binding fragment in the composition decreases to no less than 95% (e.g., no less than 98%) following storage at 2-8 °C for 12 months. In some embodiments, an amount of HMW species of the humanized anti-CD40 antibody or antigen-binding fragment in the composition increases to no more than 5% (e.g., no more than 2%) following storage at 2-8 ° C for 12 months.

### Formulation C

Formulation C: 200 mg/mL KPL-404 in 50 mM acetate, 100 mM arginine, 100 mM glutamate, 0.02% polysorbate 20, pH 5.4.

A pharmaceutical composition of the disclosure may also include modifications relative to Formulation C. For example, the pharmaceutical composition can include polysorbate 20, acetate, arginine, and glutamate in amounts that differ from Formulation C. For example, the pharmaceutical composition can include from about 5 mM to about 100 mM (e.g., about 10 mM, 20 mM, 30 mM, 40 mM, 50 mM, 60 mM, 70 mM, 80 mM, 90 mM, or 100 mM) acetate, such as about 50 mM acetate. The pharmaceutical composition can include from about 1 mM to about 200 mM (e.g., about 1 mM, 2 mM, 3 mM, 4 mM. 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) arginine, such as about 50 mM or about 100 mM arginine. The pharmaceutical composition can include from about 1 mM to about 200 mM (e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM) glutamate, such as about 50 mM or about 100 mM glutamate.

In some embodiments, the pharmaceutical composition includes from about 0.001% to about 0.1% (e.g., about 0.005% to 0.05%, e.g., about 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20, such as about 0.01% polysorbate 20. In some embodiments, the pharmaceutical composition includes about 0.01% polysorbate 20. In some embodiments, the pharmaceutical composition includes about 0.02% polysorbate 20.

The pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) arginine, from about 1 mM to about 200 mM (e.g., about 50 mM to about 150 mM, e.g., about 75 mM to about 125 mM, e.g., about 90 mM to about 110 mM, e.g., about 95 mM to about 105 mM, e.g., about 1 mM, 2 mM, 3 mM, 4 mM, 5 mM, 10 mM, 20 mM, 30 mM, 40 mM, 50 mM, 60 mM, 70 mM, 80 mM, 90 mM, 100 mM, 110 mM, 120 mM, 130 mM, 140 mM, 150 mM, 160 mM, 170 mM, 180 mM, 190 mM, or 200 mM, e.g., about 100 mM) glutamate, and from about 0.001% to about 0.1% (e.g., 0.001%, about 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20.

The pharmaceutical composition can include about 50 mM acetate, about 100 mM arginine, about 100 mM glutamate, and about 0.01% polysorbate 20. The pharmaceutical composition can include about 50 mM acetate, about 100 mM arginine, about 100 mM glutamate, and about 0.02% polysorbate 20. In some embodiments, the pharmaceutical composition includes about 50 mM acetate, about 50 mM arginine, 50 mM glutamate, and about 0.01% polysorbate 20. In some embodiments, the pharmaceutical composition includes about 50 mM acetate, about 50 mM arginine, 50 mM glutamate, and about 0.02% polysorbate 20. In some embodiments, the pharmaceutical composition includes about 50 mM acetate, about 100 mM arginine, 50 mM glutamate, and about 0.01% polysorbate 20. In some embodiments, the pharmaceutical composition includes about 50 mM acetate, about 100 mM arginine, 50 mM glutamate, and about 0.02% polysorbate 20. The pharmaceutical composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

The pharmaceutical composition can include, e.g., an amount of an anti-CD40 antibody or antigen-binding fragment thereof described herein (e.g., KPL-404), such as, e.g., about 100 mg/mL to about 300 mg/mL, e.g., about 100 mg/mL to about 250 mg/mL, about 150 mg/mL to about 250 mg/mL, or about 100 mg/mL to about 200 mg/mL (e.g., about 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL and 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL).

### Formulation A

Formulation A: 200 mg/mL KPL-404 in 50 mM acetate, 7% (w/v) sucrose, 0.01% polysorbate 20, pH 5.4.

A pharmaceutical composition of the disclosure may also include modifications relative to Formulation A. For example, the pharmaceutical composition can include polysorbate 20, acetate, and sucrose. For example, the pharmaceutical composition can include from about 5 mM to about 100 mM (e.g., about 10 mM, 20 mM, 30 mM, 40 mM, 50 mM, 60 mM, 70 mM, 80 mM, 90 mM, or 100 mM) acetate, such as about 50 mM acetate. The pharmaceutical composition can include from about 1% (w/v) to about 10% (w/v), e.g., 1%, 2%, 3%, 4%, 5%, 6%, 7%, 8%, 9%, or 10% (w/v), sucrose, such as about 7% (w/v) sucrose. The pharmaceutical composition can include from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20, such as about 0.01% or about 0.02% polysorbate 20.

The pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 1% (w/v) to about 10% (w/v), e.g., 1%, 2%, 3%, 4%, 5%, 6%, 7%, 8%, 9%, or 10% (w/v), e.g., about 7%, sucrose, and from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01%) polysorbate 20.

The pharmaceutical composition can include about 50 mM acetate, about 7% (w/v) sucrose, and about 0.01% polysorbate 20. The pharmaceutical composition can include about 50 mM acetate, about 7% (w/v) sucrose, and about 0.02% polysorbate 20.

The pharmaceutical composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

The pharmaceutical composition can include, e.g., an amount of an anti-CD40 antibody or antigen-binding fragment thereof described herein (e.g., KPL-404), such as, e.g., about 100 mg/mL to about 300 mg/mL, e.g., about 100 mg/mL to about 250 mg/mL, about 150 mg/mL to about 250 mg/mL, or about 100 mg/mL to about 200 mg/mL (e.g., about 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL and 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL).

### Formulation B

Formulation B: 200 mg/mL KPL-404 in 50 mM acetate, 2.5% (w/v) sorbitol, 70 mM NaCl, 0.01% polysorbate 20, pH 5.4.

A pharmaceutical composition of the disclosure may also include modifications relative to Formulation B. For example, the pharmaceutical composition can include polysorbate 20, acetate, sorbitol, and sodium chloride. For example, the pharmaceutical composition can include from about 5 mM to about 100 mM (e.g., about 10 mM, 20 mM, 30 mM, 40 mM, 50 mM, 60 mM, 70 mM, 80 mM, 90 mM, or 100 mM) acetate, such as about 50 mM acetate. The pharmaceutical composition can include from about 0.1% (w/v) sorbitol to about 5% (w/v), e.g., 0.2%, 0.3%, 0.4%, 0.5%, 0.6%, 0.7%, 0.8%, 0.9%, 1%, 1.5%, 2%, 2.5%, 3%, 3.5%, 4%, 4.5%, or 5% (w/v), sorbitol, such as about 2.5% (w/v) sorbitol. The pharmaceutical composition can include from about 1 mM to about 100 mM (e.g., about 1 mM, 2 mM, 3 mM, 4 mM, 5 mM, 10 mM, 20 mM, 30 mM, 40 mM, 50 mM, 60 mM, 70 mM, 80 mM, 90 mM, or 100 mM) sodium chloride, such as about 70 mM sodium chloride. The pharmaceutical composition can include from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) polysorbate 20, such as about 0.01% polysorbate 20 and 0.02% polysorbate 20.

The pharmaceutical composition can include from about 5 mM to about 100 mM (e.g., about 10 mM to about 90 mM, e.g., about 25 mM to about 75 mM, e.g., about 40 mM to about 60 mM, e.g., about 45 mM to about 55 mM, e.g., about 5 mM, 10 mM, 20 mM, 30 mM, 40 mM, 50 mM, 60 mM, 70 mM, 80 mM, 90 mM, or 100 mM, e.g., about 50 mM) acetate, from about 0.1% (w/v) sorbitol to about 5% (w/v) ( e.g., 0.2%, 0.3%, 0.4%, 0.5%, 0.6%, 0.7%, 0.8%, 0.9%, 1%, 2%, 3%, 4%, or 5% (w/v), e.g., about 2.5%) sorbitol, from about 1 mM to about 100 mM (e.g., about 10 mM to about 90 mM, e.g., about 50 mM to about 90 mM, e.g., about 60 mM to about 80 mM, e.g., about 65 mM to about 75 mM, e.g., about 5 mM, 1 mM, 2 mM, 3 mM, 4 mM, 5 mM, 10 mM, 20 mM, 30 mM, 40 mM, 50 mM, 60 mM, 70 mM, 80 mM, 90 mM, or 100 mM, e.g., about 70 mM) sodium chloride, and from about 0.001% to about 0.1% (e.g., 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%, e.g., about 0.01% or about 0.02%) polysorbate 20.

The pharmaceutical composition can include about 50 mM acetate, about 2.5% (w/v) sorbitol, about 70 mM NaCl, and about 0.01% polysorbate 20. The pharmaceutical composition can include about 50 mM acetate, about 2.5% (w/v) sorbitol, about 70 mM NaCl, and about 0.02% polysorbate 20.

The pharmaceutical composition may have a pH of from about 5.0 to about 8.0, e.g., about 5.4 to about 6.5; e.g., about 5.4 to about 5.75; e.g., 5.4 to about 5.6; e.g, 5.0, 5.1, 5.2, 5.3, 5.4, 5.5, 5.6, 5.7, 5.8, 5.9 and 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9 and 8.0. For example, the pH may be about 5.4.

The pharmaceutical composition can include, e.g., an amount of an anti-CD40 antibody or antigen-binding fragment thereof described herein (e.g., KPL-404), such as, e.g., about 100 mg/mL to about 300 mg/mL, e.g., about 100 mg/mL to about 250 mg/mL, about 150 mg/mL to about 250 mg/mL, or about 100 mg/mL to about 200 mg/mL (e.g., about 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL and 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300 mg/mL).

### Routes of Administration

A composition of the present disclosure can be formulated for administration by a variety of methods known in the art. As will be appreciated by the skilled artisan, the route and/or mode of administration will vary depending upon the desired results. Administration may be parenteral, intravenous, intrathecal, subcutaneous, oral, topical, local, intramuscular, intradermal, transdermal, subdermal, transbuccal, rectal, spinal, or epidermal. Intravenous delivery by continuous infusion is one exemplary method for administering the present antibodies or antigen-binding fragments thereof.

To administer the present agent by certain routes of administration, it may be necessary to coat the agent with, or co-administer the agent with, a material to prevent its inactivation. For example, the agent may be administered to a subject in an appropriate carrier, for example, liposomes, or a diluent. Pharmaceutically acceptable diluents include saline and aqueous buffer solutions.

Parenteral administration can include modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrastemal injection and infusion.

### Indications

The pharmaceutical compositions containing an antibody or antigen-binding fragment as described herein have *in vitro* and *in vivo* therapeutic, prophylactic, and/or diagnostic utilities. The antibodies or antigen-binding fragments thereof can be administered in a subject, as part of an *in vivo* (e.g., therapeutic or prophylactic) protocol. For *in vivo* embodiments, the contacting step includes administering an anti-CD40 antibody or portion thereof to the subject under conditions effective to permit binding of the antibody or antigen-binding fragment thereof to CD40 in the subject. The antibodies or antigen-binding fragments thereof can be administered to reduce the likelihood of, or increase the duration prior to, transplant rejection, to induce immunosuppression, or to treat an immune (e.g., an autoimmune) disorder.

In one aspect, the pharmaceutical compositions described herein can be uses in suppressing the immune system in a human subject, or for use in the manufacture of a medicament for suppressing the immune system in a human subject. The pharmaceutical compositions described herein may be used in any situation in which immunosuppression is desired (e.g., transplant rejection or autoimmune disorders). These antibodies are particularly useful for treating transplant rejection, e.g., reducing the likelihood that a particular transplant is rejected by the host or increasing the time before rejection takes place.

The pharmaceutical compositions described herein can also be used to treat autoimmune disorders. In one embodiment, the autoimmune disorder may be associated with or caused by the presence of an autoantibody.

In another embodiment, the pharmaceutical composition can be used in the treatment of various disorders associated with the expression of CD40.

A disorder may be any condition that would benefit from treatment with the present antibody or its fragment. This includes chronic and acute disorders or diseases including those pathological conditions that predispose the mammal to the disorder in question. Non-limiting examples of disorders to be treated herein include autoimmune diseases, immunologic disorders, inflammatory disorders, and cancer

### Compositions and Kits

The disclosure also features compositions and kits containing materials useful for the treatment of a condition or disorder described herein. The composition or kit may include a container including a pharmaceutical composition as described herein (e.g., one of Formulation C, A, or B containing an amount of an anti-CD40 antibody or antigen-binding fragment thereof, such as, e.g., about 100 mg/mL to about 300 mg/mL), and optionally, a label. Suitable containers include, for example, bottles, vials, syringes, prefilled syringes, autoinjectors and test tubes. The containers may be formed from a variety of materials such as glass or plastic. The container holds a pharmaceutical composition as described herein, e.g., that is effective for treating the condition, and may have a sterile access port. For example, the container may be an intravenous solution bag or a vial having a stopper pierceable by a hypodermic injection needle. The label on or associated with the container indicates that the composition is used for treating the condition of choice. The kit may further include a second container with a pharmaceutically acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use.

The kit or composition can contain an antibody or antigen-binding fragment thereof as described herein formulated to about 100 mg/mL to about 300 mg/mL. In one embodiment, the composition is formulated to about 200 mg/mL of the antibody or antigen-binding fragment thereof. The antibody or antigen-binding fragment thereof may be at a concentration of from about 0.01 mg/mL to about 300 mg/mL (e.g., 0.02 mg/mL, 0.03 mg/mL, 0.04 mg/mL, 0.05 mg/mL, 0.06 mg/mL, 0.07 mg/mL, 0.08 mg/mL, 0.09 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL, 2 mg/mL, 3 mg/mL, 4 mg/mL, 5 mg/mL, 6 mg/mL, 7 mg/mL, 8 mg/mL, 9 mg/mL, or 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, 200 mg/mL, 205 mg/mL, 210 mg/mL, 215 mg/mL, 220 mg/mL, 225 mg/mL, 230 mg/mL, 235 mg/mL, 240 mg/mL, 245 mg/mL, 250 mg/mL, 255 mg/mL, 260 mg/mL, 265 mg/mL, 270 mg/mL, 275 mg/mL, 280 mg/mL, 285 mg/mL, 290 mg/mL, 295 mg/mL, or 300, such as at a concentration of about 200 mg/mL.

The composition may be formulated in a single use vial with about 0.5 mL to about 2.0 mL (e.g., 1.0 mL or 2.0 mL) extractable volume. The composition may also be formulated in a prefilled syringe with about 0.5 mL to about 2.0 mL (e.g., 1.0 mL or 2.0 mL) injectable volume. The composition may also be formulated in an autoinjector with about 0.5 mL to about 2.0 mL (e.g., 1.0 mL or 2.0 mL) injectable volume.

The composition can be formulated in a volume of about 0.1 mL to about 10.0 mL (e.g., about 0.5 to about 5 mL, e.g., about 1.0mL to about 2.0 mL, e.g., 0.1 mL, 0.2 mL, 0.3 mL, 0.4 mL, 0.5 mL, 0.6 mL, 0.7 mL, 0.8 mL, 0.9 mL, 1.0 mL, 1.1 mL, 1.2 mL, 1.3 mL, 1.4 mL, 1.5 mL, 1.6 mL, 1.7 mL, 1.8 mL, 1.9 mL, 2.0 mL, 3.0 mL, 4.0 mL, 5.0 mL, 6.0 mL, 7.0 mL, 8.0 mL, 9.0 mL or 10.0 mL), such as a volume of about 2.0 mL.

Additional components that may be included in a kit are, for example, instructions for use; other reagents, a therapeutic agent, or an agent useful for coupling an antibody to a label or therapeutic agent, or other materials for preparing the antibody for administration; pharmaceutically acceptable carriers; and devices or other materials for administration to a subject.

### Examples

The following examples of specific aspects for carrying out the present disclosure are offered for illustrative purposes only and are not intended to limit the scope of the present disclosure in any way.

### Example 1

Described herein are studies that were performed to assess formulations that would allow for an increase in the concentration of KPL-404 to at least 100 mg/mL.

Unless otherwise noted, the candidate formulations described herein include KPL-404 and the following excipients:
**Formulation 1:** 20 mM sodium phosphate, 200 mM glycine, 0.01% polysorbate 20 (PS20), pH 6.5
**Formulation 2:** 50 mM sodium Acetate/NaAcetate, 200 mM glycine 0.01% PS20, pH 5.5
**Formulation A:** 50 mM Acetate/NaAcetate, 7%(w/v) sucrose, 0.01% PS20, pH 5.4
**Formulation B:** 50 mM Acetate/NaAcetate, 2.5%(w/v) sorbitol, 70 mM NaCl, 0.01% PS20, pH 5.4
**Formulation C:** 50 mM Acetate/NaAcetate, 100 mM Arginine, 100 mM Glutamate, 0.01% PS20, pH 5.4

Formulation C was selected as the planned formulation for 200 mg/mL KPL-404 formulation for drug substance (DS) and drug product (DP) with 0.02% PS20, instead of 0.01% PS20.

### SCREENING STUDIES

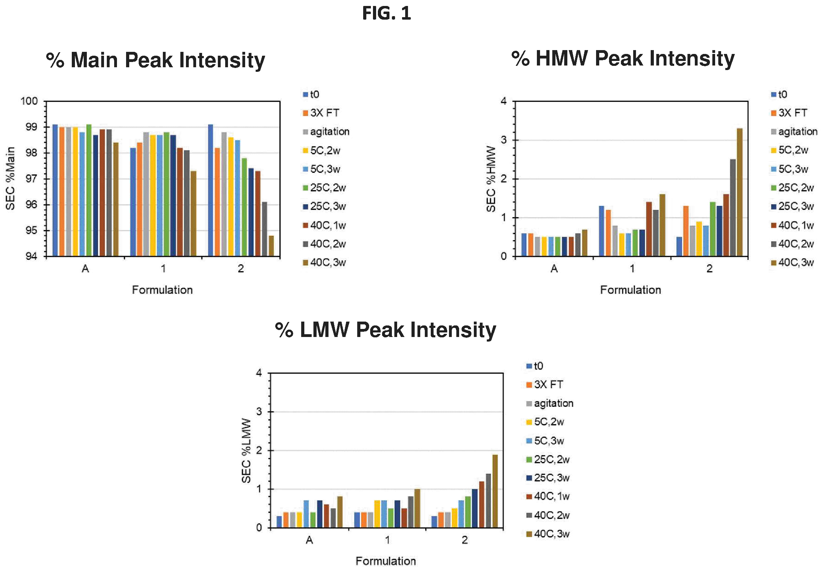

To evaluate the stability of KPL-404 in a formulation screen, KPL-404 was exchanged into 12 different buffers, including Formulations A (pH 5.6), 1 and 2. Freeze/Thaw (F/T) stress was assessed, cycling samples between -70C and ambient temperature three times. Agitation stress was also assessed by shaking at 200 rpm for 48 hours in the upright position. A short-term stability study was conducted, with samples stored at 2-8°C, 25°C, or at 40°C for up to 3 weeks. The SEC results show that the excipient does play an important role in ensuring KPL-404 stability following F/T and agitation stress, with glycine Formulations 1 and 2 being most susceptible to degradation, i.e. loss of %Main species, or increase either in %HMW or %LMW species. KPL-404 was stable in Formulation A, which included sucrose as a stabilizing agent. SEC results for screened formuations A, 1 and 2 are presented graphically in FIG 1. There are clear differences among the formulations, with Formulation 2 being the least stable showing loss of main and increase of both HMW and LMW species. Formulation 1 showed increases in HMW species at 40°C and was unstable during F/T stress and agitation tests. Formulation A was the lead candidate in these screening studies and was selected for further development studies.

### DEVELOPMENT STUDIES

Following the screening studies, subsequent studies were performed in three candidate formulations that were identified as part of the development studies:

KPL-404 was formulated at 200 mg/mL in each of the three candidate formulations (Formulations A-C) by UF/DF and set on stability in a vial presentation.

KPL-404 was highly concentrated by UF/DF until the flux went to zero. The concentrations that were achieved were:
**272 mg/mL KPL-404 in the Formulation A:** (50 mM Acetate, 7%(w/v) sucrose, 0.001% PS20, pH 5.4)
**274 mg/mL KPL-404 in Formulation B:** 50 mM Acetate, 2.5%(w/v) sorbitol, 70 mM NaCl, 0.001% PS20, pH 5.4
**291 mg/mL KPL-404 in Formulation C:** 50 mM Acetate, 100 mM Arg/100 mM Glu, 0.001% PS20, pH 5.4

Samples of each of these were characterized externally using biophysical techniques to characterize self-association as a function of protein concentrations. Drug substance material used to prepare these formulations was initially manufactured with PS20 at 0.001% . Separately, formulations were spiked with 10% (w/v) PS20 to achieve 0.01% PS20 and were also analyzed.

In parallel, CG-MALS testing was performed on formulations with PS20 adjusted to 0.01%.

Viscosity and Instron testing were performed as a function of protein concentration to assess syringeability.

From these characterization studies, it was concluded that KPL-404 could achieve a concentration of up to 292 mg/mL in Formulation C. Abbreviated stability studies were performed at 220 mg/mL each of Formulations A and B, and at 235 mg/mL in Formulation C.

We selected Formulation C at 200 mg/mL KPL-404 for both drug substance and for drug product based on its improved characteristics relative to Formulations A and B.

### MATERIALS

### Material Generation for Assessing High Concentration of KPL-404

### Initial Testing

Non-GMP drug substance was diluted into each of the three buffers, followed by concentration to 200 mg/mL. It was expected that the polysorbate 20 present in the DS would co-concentrate with KPL-404 in the centrifugal unit.

### Material Generation for Stability Studies

### UFDF Testing:

To generate load material for each ProA cycle, drug substance was thawed in a 25 °C water bath and adjusted using ProA equilibration buffer to a target 10 g/L. The conditioned ProA load material was 0.2 µm filtered prior to loading the column. Each Pro A eluate was neutralized to target pH 5.00, 0.2 µm filtered, and stored at ≤-65 °C. A total of 4 cycles were performed to generate 68 grams of UFDF load material for high concentration studies.

### Material Generation for UFDF Confirmation:

To generate load material for each ProA cycle, drug substance was thawed at 2-8°C overnight and adjusted using ProA equilibration buffer to a target 10 g/L. The conditioned ProA load material was 0.2 µm filtered prior to loading the column. Each Pro A eluate was 0.2 µm filtered and stored at ≤-65 °C. A total of 3 cycles were performed to generate 54 grams of UFDF load material for high concentration studies.

For each run, neutralized ProA eluate was thawed at 2-8 °C, 0.2 µm filtered, and used as UFDF load. The starting material was concentrated to a target 50 g/L, diafiltered into the relevant buffer and then concentrated until a prohibitive drop in flux occurred or the retentate showed visual deterioration. During processing, when a target of 200 g/L was reached, 40 mL of the in-process pool was sampled for drug product processing. The remaining pool was then further concentrated while adjusting feed flow rate and transmembrane pressure to ensure equipment capacity was not exceeded at high concentration and high pressure.

A system rinse using 1x to 1.5x the holdup volume was performed to allow for a process step mass balance calculation. A PS20 stock solution of 10% w/v was prepared in each respective formulation buffer and spiked into both the target 200 g/L sample to a target 0.01% PS20. Final 0.2 µm filtration was performed using 3.5 cm² PES filters from Sartorius. Each pool was filtered separately, and the pressure monitored. The remainder of the drug substance pools were handled in accordance with the DP stability study to ensure compliance with that study's objectives.

### RESULTS AND DISCUSSION

### Effect of formulations A-C on > 200 mg/mL KPL-404

Small scale UF/DF was performed to generate the following samples, which were concentrated until the tangential flow stopped. These were considered to be at their threshold of solubility:
**Formulation A** (sucrose-based, Ph1 formulation): 50 mM Acetate, 7%(w/v) sucrose, 0.01% PS20, pH 5.4 - achieved 212.6 mg/mL KPL-404
**Formulation B** (sorbitol-based): 50 mM Acetate, 2.5%(w/v) sorbitol, 70 mM NaCl, 0.01% PS20, pH 5.4 - achieved 195.5 mg/mL KPL-404
**Formulation C** (Arg/Glu-based): 50 mM Acetate, 100 mM Arg/100 mM Glu, 0.01% PS20, pH 5.4 - achieved 214.1 mg/mL KPL-404

The study used KPL-404 drug substance (i.e., an antibody having a heavy chain variable region with the amino acid sequence set forth in SEQ ID NO: 9, and a light chain variable region with the amino acid sequence set forth in SEQ ID NO: 10) that was processed over a Protein-A column to remove all residual buffer components including PS20, followed by ultrafiltration/diafiltration (UFDF) into each of Formulation A, Formulation B, or Formulation C, at 200 mg/mL. The formulations were then further concentrated up to ~250 mg/mL. To all of these, PS20 was added to a target of 0.01% and then filtered.

### Biophysical characterization

### Malvern Panalytical Studies

Formulations were studied by a variety of biophysical techniques, including dynamic light scattering, static light scattering (B22 and CIMax), electrophoretic light scattering (Zeff), thermodynamic stability by differential scanning calorimetry (melting transitions), injectability by microcapillary viscometry and rheology, and nanoparticle tracking analysis.

An initial set of formulations were characterized at 200 mg/mL as shown in **Table 2.** It was discovered that Formulation C lacked PS20 after testing had been completed.

**Table 2: Biophysical Stability Profiles of 200 mg/mL KPL-404 Formulations**

| **Formulation** | **Developability** | | | | | | **Injectability** | | **Manufacturability** | | | | **SVPs** |
|---|---|---|---|---|---|---|---|---|---|---|---|---|---|
| | **B₂₂** | **R_{S}** | **k_{D}** | **k_{D}*** | **Z_{eff}** | **C_{IMax}** | **η₁₀₀** | **η_{stock}** | **T₀** | **Tₘ₁** | **Tₘ₂** | **Tₘ₃** | **C_{sµp}** |
| A^{‡} | 8.9e-5 | 5.1 | -0.65 | 5.79 | 1.4 | 113 | 2.296 | 21.22 | 57.4 | 65.7 | 72.1 | 75.2 | 5.15e7 |
| B^{‡} | 4.4e-5 | 5.3 | -2.26 | 3.49 | 2.0 | 113 | 2.071 | 14.45 | 56.5 | 64.9 | 71.5 | 74.1 | 3.85e8 |
| C^{‡} | 1.8e-5 | 5.4 | -1.91 | 3.08 | 1.8 | 108 | 2.035 | 14.79 | 56.5 | 64.6 | 71.5 | 74.5 | 1.51e9 |

| | | | | | | | | | | | | | |
|---|---|---|---|---|---|---|---|---|---|---|---|---|---|
| ^{‡}With ~0.001% polysorbate 20; *viscosity-corrected k_{D} | | | | | | | | | | | | | |

**Table 3: Biophysical Stability Profiles of High Concentration KPL-404 Formulations**

| **Formulation** | **Developability** | | | | | | **Injectability** | | **Manufacturability** | | | | **SVPs** |
|---|---|---|---|---|---|---|---|---|---|---|---|---|---|
| | **B₂₂** | **R_{S}** | **k_{D}** | **k_{D}*** | **Z_{eff}** | **C_{IMax}** | **η₁₀₀** | **η_{stock}** | **T₀** | **Tₘ₁** | **Tₘ₂** | **Tₘ₃** | **C_{sµp}** |
| A^{†} | 6.9e-5 | 5.1 | -2.43 | 3.41 | N.T. | 113 | 2.630 | 91.10 | 56.8 | 65.4 | 72.5 | 75.3 | 3.05e8 |
| B^{†} | 4.1 e-5 | 5.4 | -3.08 | 9.60 | N.T. | 113 | 3.100 | 48.20 | 53.8 | 67.2 | 73.0 | | 9.47e8 |
| C^{†} | 7.9e-5 | 5.4 | -1.01 | 19.92 | N.T. | 108 | 2.330 | 67.10 | 56.3 | 67.7 | 73.7 | | 7.42e8 |

| | | | | | | | | | | | | | |
|---|---|---|---|---|---|---|---|---|---|---|---|---|---|
| ^{†}With ~0.02% polysorbate 20; *viscosity-corrected k_{D} | | | | | | | | | | | | | |

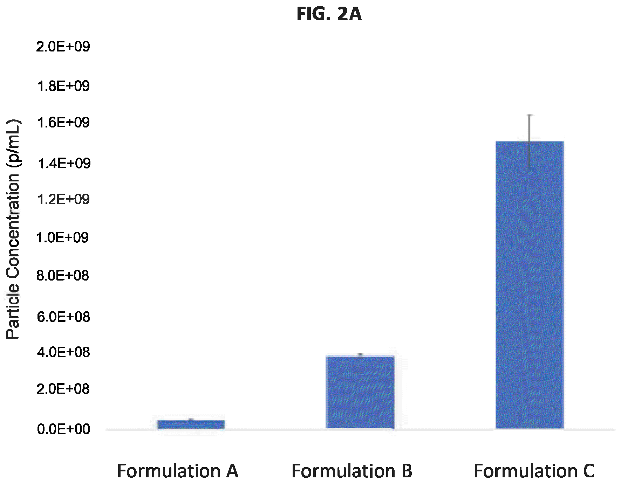

With the formulations containing nominal levels of PS20, Formulation C had the lowest colloidal stability based on kD value. Similarly, Formulation C had the highest concentration of subvisible particles (C_{sµp}) compared to Formulations A and B **(****FIG. 2A****).**

Thermal analysis indicated similar thermal stability for aggregation onset and for each of the three observed thermal transitions, with Formulation A being higher in stability due to sucrose, in comparison to Formulations B and C which were essentially equivalent.

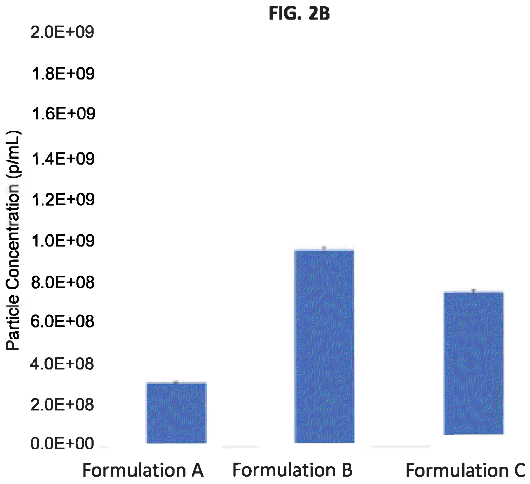

Biophysical characterization studies were repeated following generation of high concentration (> 200 mg/mL) KPL-404 formulations, including 0.02% PS20 in Formulation C, **Table 3.** When 0.02% PS20 was in the formulations, Formulation C had the highest colloidal stability based on kD value. Similarly, Formulation C had a lower concentration of subvisible particles (C_{sµp}) compared to Formulation B **(****FIG. 2B****).**

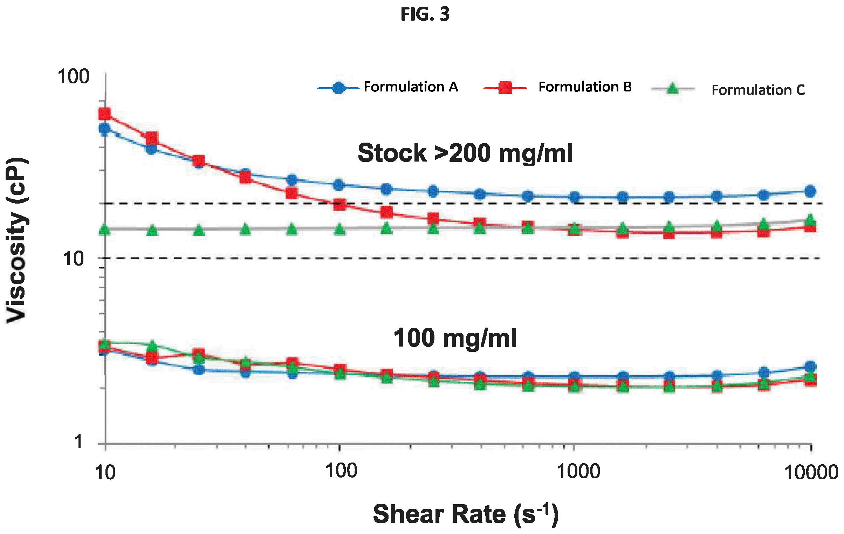

Rheological assessment of viscosity as a function of shear rate showed different behavior at 100 mg/mL and > 200 mg/mL **(****FIG. 3****).**

At 100 mg/mL, all formulations demonstrated Newtonian behavior with similar viscosities as a function of shear rate. However, at > 200 mg/mL a common non-Newtonian "shear-thinning" effect for Formulations A and B, but a static invariant viscosity vs shear rate is observed for Formulation C. This suggests the Arg/Glu excipients in Formulation C inhibit self-association at high concentration, whereas low energy self-associations were present at low shear-rates for Formulations A and B that were disrupted as the shear rate increases.

This behavior is consistent with a change in mechanism of colloidal stability above 250 mg/mL, wherein protein self-association becomes dominant rather than the hard sphere mixture model that is characteristic of a dilute protein solution for KPL-404 in Formulations A and B. Conversely, for KPL-404 in Formulation C, the absence of shear thinning at elevated concentrations translates to an exponential increase in viscosity as a function of protein concentration up to 292mg/mL. The combination of arginine and glutamate stabilizes KPL-404 against self-association via a different mechanism than sucrose in Formulation A or sorbitol/NaCl in Formulation B.

In summary, biophysical characterization of KPL-404 formulations showed differences in mechanism of stabilization, with the sucrose Formulation A being less optimal than either Formulation B or C. Data suggest Formulation C may be most effective at limiting self-association at elevated concentrations through a combination of Arg/Glu and PS20.

### Biophysical Studies

Concentration-gradient multiangle light scattering (CG-MALS) was employed to assess oligomeric behavior of KPL-404 over a range of concentrations. Individual samples were prepared at different concentrations and analyzed by static light scattering (SLS).

Wyatt technologies has implemented CG-MALS by merging an autosampler that generates a concentration gradient from two solutions (from a high concentration protein formulation and matched placebo) in conjunction with in-line sample analysis.

KPL-404 formulations and matching formulation buffers were filtered using 0.22 µm syringe filters. Fifteen concentrations were analyzed per formulation, with concentrations confirmed by UV Absorbance.
Fitting of data to reversible/irreversible association models were performed using Wyatt Calypso software (144 kDa monomer).

### CG-MALS results for Formulation A

No reversible associations could be modeled with the highest concentration points included. The inability to fit reversible oligomers may be due to repulsive interactions at higher concentrations dominating and masking the underlying attractive interactions

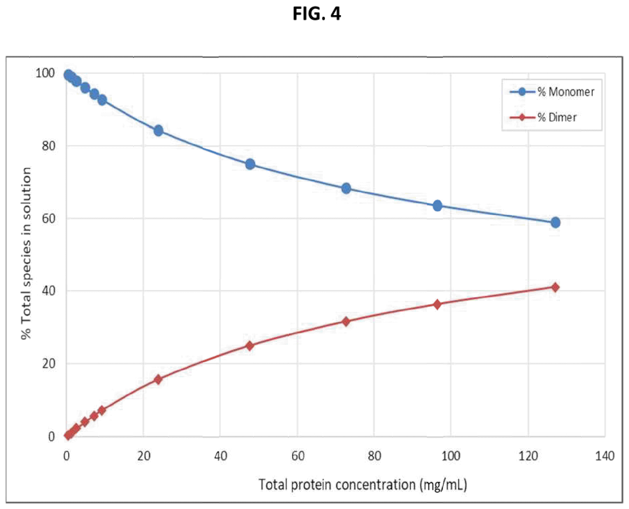

Due to strong repulsive interactions masking underlying attractive interactions, the highest concentration we could fit with the reversible associations model was ~127 mg/mL with reversible dimer. Data up to ~127 mg/mL could be modeled with reversible dimer. Concentration of reversible dimer did not exceed the monomer within the concentration range that we could fit. Reversible dimer was present even at the lowest concentration (~0.5 mg/mL); see **FIG. 4****.**

### CG-MALS results for Formulation B

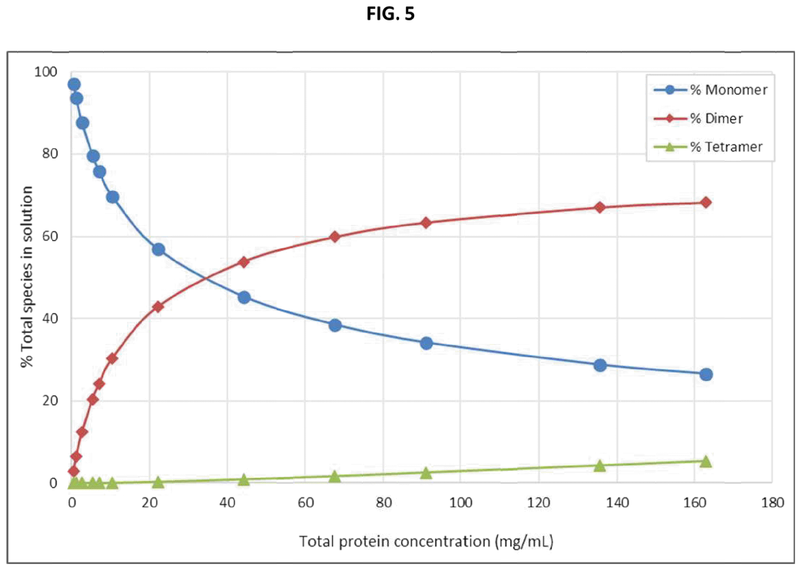

Similarly, no reversible associations could be modeled with the highest concentration points included. Due to strong repulsive interactions masking underlying attractive interactions, the highest concentration that could be fit with the reversible associations model was ~163 mg/mL with reversible dimer and hexamer **(****FIG. 5****).**

The percentage of reversible oligomers exceed the monomer at ~35 mg/mL. Reversible dimer was present even at the lowest concentration (~0.5 mg/mL),

### CG-MALS results for Formulation C

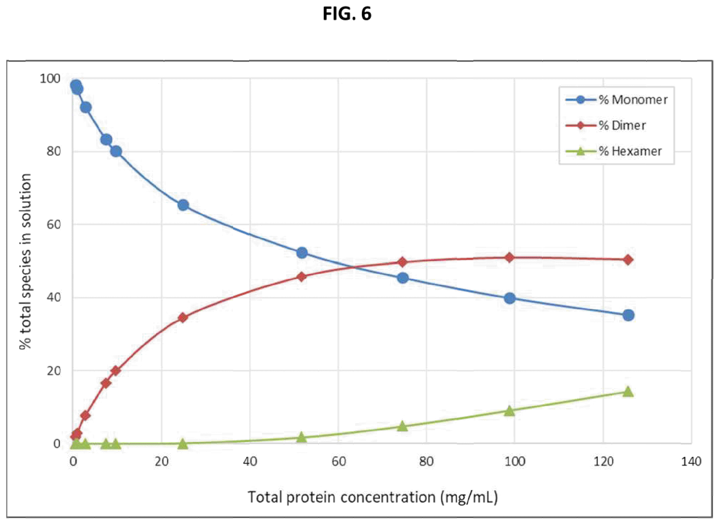

Similarly, no reversible associations could be modeled with the highest concentration points included. Due to strong repulsive interactions masking underlying attractive interactions, the highest concentration we could fit with the reversible associations model was ~126 mg/mL with reversible dimer and hexamer **(****FIG. 6****).**

The percentage of reversible oligomers exceed the monomer at ~60 mg/mL. Reversible hexamer starts to form at ~25 mg/mL. Approximately 2% dimer was present even at lowest total protein concentration

In summary, CG-MALS demonstrates KPL-404 readily forms reversible dimers and higher ordered oligomer that are not detected by SEC (which dilutes to ~1 mg/mL prior to injection).

Results were generally consistent with a hard sphere repulsive behavior, with underlying attractive behavior in all formulations as indicated by the need to include reversible associations in global analysis.

Formulation A was more effective at inhibiting formation of reversible oligomers below 125 mg/mL, but data were not fittable at higher concentrations.

Formulation B was the least stable with respect to oligomerization, with reversible oligomers becoming the dominant species at ~35 mg/mL.

Formulation C shows oligomers become the dominant species at > 60 mg/mL, and has lower viscosity compared to formulation A, which is consistent with lower energy self-association at elevated concentrations for Formulation C.

These data suggest that KPL-404 is less prone to aggregation not only at concentrations exceeding 200 mg/mL, but also at lower concentrations, e.g., 150-200 mg/mL. Both the rheology and light scattering data suggest that KPL-404 self-association tends to increase with increasing concentration. The shear-thinning data reveal a notable and unexpected difference between the self-association dynamics of formulation C from the other formulations, suggesting that arginine and glutamate affect these interactions via a different mechanism. The light scattering data demonstrate that KPL-404 is predominantly a monomer in Formulations A, B and C below ~100 mg/mL. At higher concentrations, however, these data suggest that Formulation C has a superior ratio of reversible dimer to non-reversible dimer compared to Formulations A and B.

### Syringeability characterization.

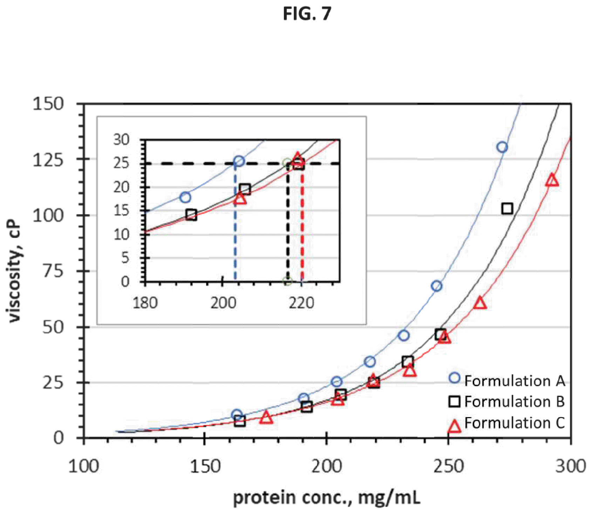

Viscosity was assessed as a function of variable concentration for each of the three formulations, **Table 4** and **FIG. 7****.**

Viscosity varied exponentially with respect to protein concentration. The inset in **FIG. 7** highlights the KPL-404 concentration where a given formulation crosses 25 cP, a theoretical upper limit on syringeability based on extrusion force. Formulation A with sucrose exceeded 25 cP at ~200 mg/mL, Formulations B and C exceeded 25 cP above 220 mg/mL, with Formulation C being slightly less viscous than B.

**Table 4: Conditions for KPL-914 Current Formulation PFS Stability Study.**

| **Dilution Factor** | **KPL-404-A nominal conc, mg/mL** | **Viscosity of A, cP** | **KPL-404-B nominal conc, mg/mL** | **Viscosity of B, cP** | **KPL-404-C nominal conc, mg/mL** | **Viscosity of C, cP** |
|---|---|---|---|---|---|---|
| 1.00 | 272.0 | 130.8 | 274.0 | 103.0 | 292.0 | 116.0 |
| 0.90 | 244.8 | 68.4 | 246.6 | 46.4 | 262.8 | 61.2 |
| 0.85 | 231.2 | 46.1 | 232.9 | 34.5 | 248.2 | 45.6 |
| 0.80 | 217.6 | 34.7 | 219.2 | 24.8 | 233.6 | 31.0 |
| 0.75 | 204.0 | 25.5 | 205.5 | 19.6 | 219.0 | 26.1 |
| 0.70 | 190.4 | 17.8 | 191.8 | 14.2 | 204.4 | 17.9 |
| 0.60 | 163.2 | 10.3 | 164.4 | 8.0 | 175.2 | 9.4 |

Each of Formulations A, B, and C were filled by hand into a 2.25 mL BD Neopak syringe. The same sample was tested five times with a new syringe for each test. Instron testing was performed, recording gliding forces as a function of protein concentration for each formulation, **Table 5.** To reserve sample, testing utilized the highest concentration sample of each formulation, diluting the recovered sample with buffer to generate lower concentration. The results show decreasing injection force as a function of decreasing concentration.

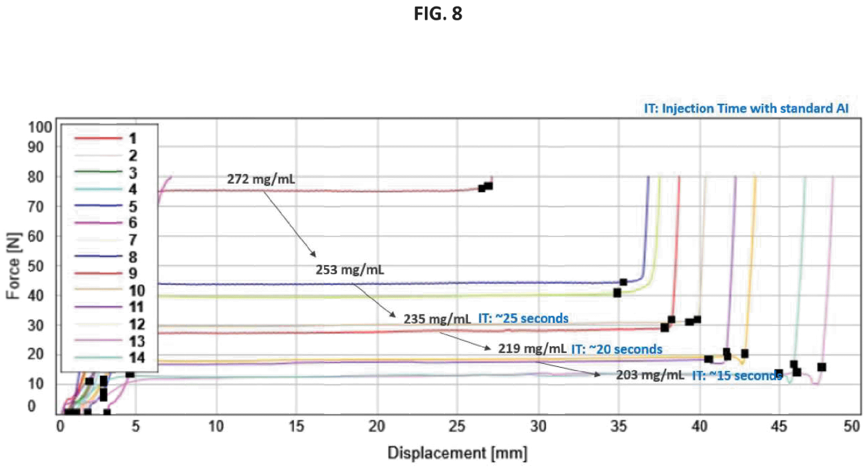

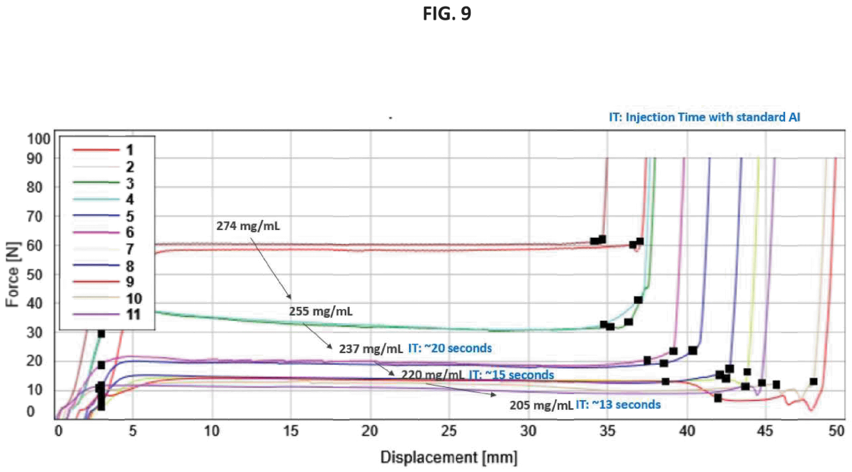

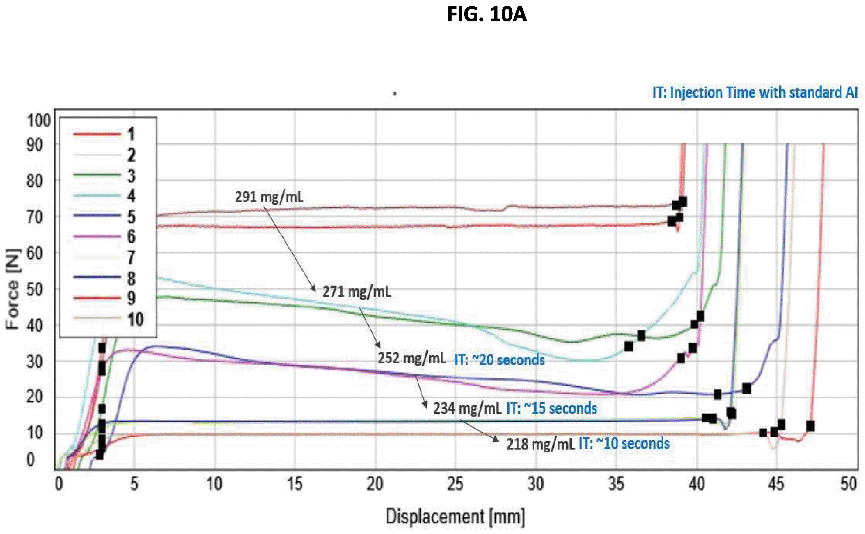

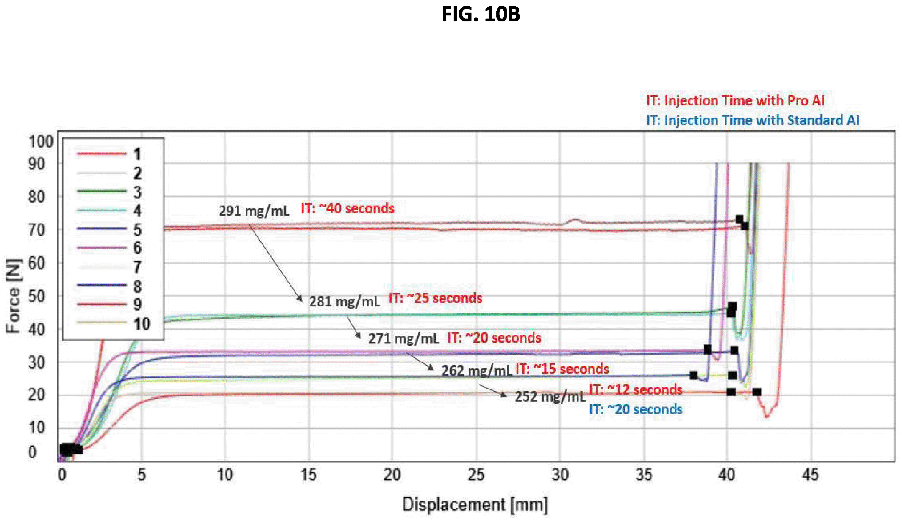

Injection times for the lowest three concentrations were recorded using manually assembled Auto-Injectors (AI) with each of the candidate formulations in a 2.25 mL BD Neopak syringe, **Table 5.** The time was established by video recording, calculating time from frame rate and the number of frames from first to last drop.
The standard Ypsomate AI was tested in all three candidates:
- Injection times (into air) for formulation A were ≥20 s at 220 mg/mL and higher and was ~15 s at 200 mg/mL.
- Injection times for formulation B were ≥20 s at 237 mg/mL and were 15 s at 220 mg/mL.
- Injection times for formulation C were ≥20 s at 252mg/mL and were 15 s at 234 mg/mL.
The Ypsomate Pro AI was tested only on formulation C:
- Injection times for formulation C were ≥20 s at 271mg/mL and were 15 s at 262 mg/mL. See **Table 5** and **FIGS. 8-10B****.**

**Table 5: Syringeability of KPL-404 High Concentration Formulation Candidates.**

| **Formulation** | **Concentration, mg/mL** | **End of Injection Gliding Force, N** | **Injection Time for Std Yposmate AI, s** | **Injection Time for Yposmate-Pro AI, s** |
|---|---|---|---|---|
| A | 272 | ~80 | N.A. | N.A. |
| | 253 | ~45 | N.A. | N.A. |
| | 235 | ~30 | ~25 | N.A. |
| | 219 | ~20 | ~20 | N.A. |
| | 203 | ~15 | ~15 | N.A. |
| B | 274 | ~60 | N.A. | N.A. |
| | 255 | ~30 | N.A. | N.A. |
| | 237 | ~20 | ~20 | N.A. |
| | 220 | ~15 | ~15 | N.A. |
| | 205 | ~10 | ~10 | N.A. |
| C | 291 | ~70 | N.A. | ~40 |
| | 281 | ~45 | N.A. | ~25 |
| | 271 | ~33 | N.A. | ~20 |
| | 262 | ~26 | N.A. | ~15 |
| | 252 | ~20 | ~20 | ~12 |
| | 234 | ~15 | ~15 | N.A. |
| | 218 | ~10 | ~10 | N.A. |

In summary, glide forces and injection times generally correlate with viscosities, but Formulation C consistently achieved higher concentrations as compared to Formulation B, with the sucrose Formulation A performing poorer than B and C.

### Stability of 200 mg/mL and > 200 mg/mL KPL-404 formulations

All three formulations were examined for stability at 200 mg/mL, which is the upper concentration limit for Formulation A based on injectability data.

Injectability data suggests that Formulation B can support 220 mg/mL. For head-to-head comparison, both Formulations A and B were put on short-term stability (3 months) at 220 mg/mL. Injectability data suggests Formulation C can support 235 mg/mL, which was likewise put on short-term stability (3 mo). The data are summarized in Tables 6-14.

**Table 6: Formulation A, 200 mg/mL stability, 5°C.**

| **Tests** | **Criteria** | **t0** | **2w,5C** | **1M,5C** | **2M,5C** | **3M,5C** | **6M,5C** | **9M,5C** | **12M,5C** |
|---|---|---|---|---|---|---|---|---|---|
| icIEF - %Acidic | Report Result | 33.4 | NT | NT | NT | 34.8 | NT | NT | NT |
| icIEF - %Main | Report Result | 50.2 | NT | NT | NT | 47.9 | NT | NT | NT |
| icIEF - %Basic | Report Result | 16.4 | NT | NT | NT | 17.3 | NT | NT | NT |
| NR CE-SDS - %IgG | Report Result | NT | NT | NT | NT | 99.0 | NT | NT | NT |
| NR CE-SDS - %Highest Impurity | Report Result | NT | NT | NT | NT | 0.5 | NT | NT | NT |
| SEC%Main | ≥95% | 98.9 | 98.6 | 98.7 | 98.5 | 98.5 | 98.2 | 98.2 | 98.1 |
| SEC%HMW | Report Result | 1.1 | 1.4 | 1.4 | 1.5 | 1.5 | 1.8 | 1.9 | 1.9 |
| SEC%LMW | Report Result | ND | ND | ND | ND | ND | ND | ND | ND |

| SVP analysis | | | | | | | | | |
|---|---|---|---|---|---|---|---|---|---|
| ≥2to<10 µm | Report Result | 31 | 21640 | 1965 | 2455 | 7485 | 6080 | 3975 | 26117 |
| ≥10 µm | Report Result | 1 | 440 | 55 | 90 | 195 | 160 | 175 | 95 |
| ≥25 µm | Report Result | 99 | 10 | 0 | 25 | 15 | 40 | 25 | 0 |

| | | | | | | | | | |
|---|---|---|---|---|---|---|---|---|---|
| NT = Not tested, ND = Not detectable (<LOD) | | | | | | | | | |

**Table 7: Formulation A, 200 mg/mL stability: agitation, stressed at 40°C, accelerated at 25°C.**

| **Tests** | **Criteria** | **t0** | **agitatio n** | **1w, 40C** | **2w, 40C** | **1M, 40C** | **1w,25C** | **2w,25C** | **1M,25C** | **2M,25C** | **3M,25C** | **6M,25C** |
|---|---|---|---|---|---|---|---|---|---|---|---|---|
| icIEF - %Acidic | Report Result | 33.4 | NT | NT | NT | 44.4 | NT | NT | NT | NT | 39.5 | NT |
| icIEF - %Main | Report Result | 50.2 | NT | NT | NT | 40.2 | NT | NT | NT | NT | 44.3 | NT |
| icIEF - %Basic | Report Result | 16.4 | NT | NT | NT | 15.3 | NT | NT | NT | NT | 16.2 | NT |
| NR CE-SDS - %IgG | Report Result | NT | NT | NT | NT | 97.8 | NT | NT | NT | NT | 98.4 | NT |
| NR CE-SDS - %Highest Impurity | Report Result | NT | NT | NT | NT | 0.7 | NT | NT | NT | NT | 0.6 | NT |
| SEC%Main | ≥95% | 98.9 | 98.7 | 97.7 | 97.3 | 96.2 | 98.5 | 98.3 | 98.2 | 97.8 | 97.6 | 97.1 |
| SEC%HMW | Report Result | 1.1 | 1.3 | 2.3 | 2.7 | 3.7 | 1.6 | 1.7 | 1.8 | 2.2 | 2.4 | 3.0 |
| SEC%LMW | Report Result | ND | ND | 0.0 | 0.0 | 0.1 | ND | ND | ND | 0.0 | 0.1 | ND |

| SVP analysis | | | | | | | | | | | | |
|---|---|---|---|---|---|---|---|---|---|---|---|---|
| ≥2to<10 µm | Report Result | 31 | 0 | 24270 | 4800 | 1685 | 5710 | 12060 | 1595 | 2100 | 11995 | |
| ≥10 µm | Report Result | 1 | 1 | 310 | 120 | 15 | 140 | 330 | 25 | 30 | 215 | |
| ≥25 µm | Report Result | 99 | 99 | 40 | 20 | 0 | 20 | 30 | 5 | 5 | 40 | |

| | | | | | | | | | | | | |
|---|---|---|---|---|---|---|---|---|---|---|---|---|
| NT = Not tested, ND = Not detectable (<LOD) | | | | | | | | | | | | |

**Table 8: Formulation A, 200 mg/mL stability, frozen at -20°C or -70°C.**

| **Tests** | **Criteria** | **t0** | **3M,-20C** | **3M,-70C** | **6M,-70C** | **9M,-70C** | **12M,-70C** |
|---|---|---|---|---|---|---|---|
| icIEF - %Acidic | Report Result | 33.4 | NT | NT | NT | 36.0 | 34.9 |
| icIEF - %Main | Report Result | 50.2 | NT | NT | NT | 47.1 | 48.2 |
| icIEF - %Basic | Report Result | 16.4 | NT | NT | NT | 16.9 | 16.9 |
| NR CE-SDS - %IgG | Report Result | NT | NT | NT | NT | 99.0 | 98.9 |
| NR CE-SDS - %Highest Impurity | Report Result | NT | NT | NT | NT | 0.5 | 0.5 |
| SEC%Main | ≥95% | 98.9 | 98.7 | 98.7 | NT | 98.8 | 98.8 |
| SEC%HMW | Report Result | 1.1 | 1.4 | 1.3 | NT | 1.2 | 1.2 |
| SEC%LMW | Report Result | ND | ND | ND | NT | ND | ND |

| SVP analysis | | | | | | | |
|---|---|---|---|---|---|---|---|
| ≥2to<10 µm | Report Result | 31 | 44720 | 54875 | NT | 178840 | 126116 |
| ≥10 µm | Report Result | 1 | 2310 | 3085 | NT | 3700 | 3725 |
| ≥25 µm | Report Result | 99 | 350 | 450 | NT | 307 | 317 |

| | | | | | | | |
|---|---|---|---|---|---|---|---|
| NT = Not tested, ND = Not detectable (<LOD) | | | | | | | |

**Table 9: Formulation B, 200 mg/mL stability, 5°C.**

| **Tests** | **Criteria** | **t0** | **2w,5C** | **1M,5C** | **2M,5C** | **3M,5C** | **6M,5C** | **9M,5C** | **12M,5C** |
|---|---|---|---|---|---|---|---|---|---|
| icIEF - %Acidic | Report Result | 34.6 | NT | NT | NT | 34.7 | NT | NT | NT |
| icIEF - %Main | Report Result | 50.9 | NT | NT | NT | 48.3 | NT | NT | NT |
| icIEF - %Basic | Report Result | 14.5 | NT | NT | NT | 17.0 | NT | NT | NT |
| NR CE-SDS - %IgG | Report Result | NT | NT | NT | NT | 99.1 | NT | NT | NT |
| NR CE-SDS - %Highest Impurity | Report Result | NT | NT | NT | NT | 0.4 | NT | NT | NT |
| SEC%Main | ≥95% | 98.9 | 98.7 | 98.6 | 98.5 | 98.5 | 98.2 | 98.2 | 98.1 |
| SEC%HMW | Report Result | 1.1 | 1.3 | 1.4 | 1.5 | 1.5 | 1.8 | 1.9 | 1.9 |
| SEC%LMW | Report Result | ND | ND | ND | ND | ND | ND | ND | ND |

| SVP analysis | | | | | | | | | |
|---|---|---|---|---|---|---|---|---|---|
| ≥2to<10 µm | Report Result | 0 | 4980 | 2665 | 1270 | 6570 | 14130 | 5835 | 9111 |
| ≥10 µm | Report Result | 1 | 40 | 110 | 40 | 160 | 345 | 165 | 275 |
| ≥25 µm | Report Result | 99 | 20 | 5 | 15 | 35 | 15 | 5 | 0 |

| | | | | | | | | | |
|---|---|---|---|---|---|---|---|---|---|
| NT = Not tested, ND = Not detectable (<LOD) | | | | | | | | | |

**Table 10: Formulation B, 200 mg/mL stability: agitation, stressed at 40°C, accelerated at 25°C.**

| **Tests** | **Criteria** | **t0** | **agitation** | **1w, 40C** | **2w, 40C** | **1M, 40C** | **1w,25C** | **2w,25C** | **1M,25C** | **2M,25C** | **3M,25C** | **6M,25C** |
|---|---|---|---|---|---|---|---|---|---|---|---|---|
| icIEF - %Acidic | Report Result | 34.6 | NT | NT | NT | 44.2 | NT | NT | NT | NT | 38.7 | NT |
| icIEF - %Main | Report Result | 50.9 | NT | NT | NT | 39.9 | NT | NT | NT | NT | 44.6 | NT |
| icIEF - %Basic | Report Result | 14.5 | NT | NT | NT | 15.9 | NT | NT | NT | NT | 16.8 | NT |
| NR CE-SDS - %IgG | Report Result | NT | NT | NT | NT | 97.9 | NT | NT | NT | NT | 98.5 | NT |
| NR CE-SDS - %Highest Impurity | Report Result | NT | NT | NT | NT | 0.6 | NT | NT | NT | NT | 0.6 | NT |
| SEC%Main | ≥95% | 98.9 | 98.5 | 97.6 | 97.2 | 96.1 | 98.4 | 98.4 | 98.1 | 97.9 | 97.6 | 97.1 |
| SEC%HMW | Report Result | 1.1 | 1.5 | 2.4 | 2.8 | 3.8 | 1.6 | 1.6 | 1.9 | 2.1 | 2.4 | 2.9 |
| SEC%LMW | Report Result | ND | ND | 0.1 | 0.1 | 0.1 | ND | ND | ND | ND | 0.0 | ND |

| SVP analysis | | | | | | | | | | | | |
|---|---|---|---|---|---|---|---|---|---|---|---|---|
| ≥2to<10 µm | Report Result | 0 | 0 | 8510 | 5990 | 1950 | 5440 | 6080 | 2365 | 1695 | 9045 | NT |
| ≥10 µm | Report Result | 1 | 2 | 170 | 190 | 35 | 210 | 110 | 100 | 30 | 385 | NT |
| ≥25 µm | Report Result | 99 | 99 | 40 | 30 | 5 | 40 | 0 | 10 | 10 | 70 | NT |

| | | | | | | | | | | | | |
|---|---|---|---|---|---|---|---|---|---|---|---|---|
| NT = Not tested, ND = Not detectable (<LOD) | | | | | | | | | | | | |

**Table 11: Formulation B, 200 mg/mL stability, frozen at -20°C or -70°C.**

| **Tests** | **Criteria** | **t0** | **3M,-20C** | **3M,-70C** | **6M,-70C** | **9M,-70C** | **12M,-70C** |
|---|---|---|---|---|---|---|---|
| icIEF - %Acidic | Report Result | 34.6 | NT | NT | NT | 35.0 | 34.9 |
| icIEF - %Main | Report Result | 50.9 | NT | NT | NT | 48.2 | 48.6 |
| icIEF - %Basic | Report Result | 14.5 | NT | NT | NT | 16.7 | 16.5 |
| NR CE-SDS - %IgG | Report Result | NT | NT | NT | NT | 99.0 | 98.9 |
| NR CE-SDS - %Highest Impurity | Report Result | NT | NT | NT | NT | 0.5 | 0.5 |
| SEC%Main | ≥95% | 98.9 | 98.5 | 98.7 | NT | 98.8 | 98.7 |
| SEC%HMW | Report Result | 1.1 | 1.5 | 1.3 | NT | 1.2 | 1.3 |
| SEC%LMW | Report Result | ND | ND | ND | NT | ND | ND |

| SVP analysis | | | | | | | |
|---|---|---|---|---|---|---|---|
| ≥2to<10 µm | Report Result | 0 | 27225 | 90130 | NT | 100040 | 169540 |
| ≥10 µm | Report Result | 1 | 1665 | 1870 | NT | 2640 | 2968 |
| ≥25 µm | Report Result | 99 | 105 | 145 | NT | 233 | 143 |

| | | | | | | | |
|---|---|---|---|---|---|---|---|
| NT = Not tested, ND = Not detectable (<LOD) | | | | | | | |

**Table 12: Formulation C, 200 mg/mL stability, 5°C.**

| **Tests** | **Criteria** | **t0** | **2w,5C** | **1M,5C** | **2M,5C** | **3M,5C** | **6M,5C** | **9M,5C** | **12M,5C** |
|---|---|---|---|---|---|---|---|---|---|
| icIEF - %Acidic | Report Result | 31.9 | NT | NT | NT | 35.6 | NT | NT | NT |
| icIEF - %Main | Report Result | 54.6 | NT | NT | NT | 47.7 | NT | NT | NT |
| icIEF - %Basic | Report Result | 13.5 | NT | NT | NT | 16.7 | NT | NT | NT |
| NR CE-SDS - %IgG | Report Result | NT | NT | NT | NT | 99.1 | NT | NT | NT |
| NR CE-SDS - %Highest Impurity | Report Result | NT | NT | NT | NT | 0.5 | NT | NT | NT |
| SEC%Main | ≥95% | 99.1 | 98.9 | 98.8 | 98.8 | 98.7 | 98.7 | 98.7 | 98.6 |
| SEC%HMW | Report Result | 0.9 | 1.1 | 1.2 | 1.3 | 1.3 | 1.3 | 1.4 | 1.4 |
| SEC%LMW | Report Result | ND | ND | ND | ND | ND | ND | ND | ND |

| SVP analysis | | | | | | | | | |
|---|---|---|---|---|---|---|---|---|---|
| ≥2to<10 µm | Report Result | 25 | 4250 | 2250 | 3570 | 10850 | 22315 | 13830 | 41865 |
| ≥10 µm | Report Result | 1 | 50 | 95 | 80 | 240 | 720 | 165 | 389 |
| ≥25 µm | Report Result | 99 | 0 | 5 | 5 | 5 | 5 | 10 | 0 |

| | | | | | | | | | |
|---|---|---|---|---|---|---|---|---|---|
| NT = Not tested, ND = Not detectable (<LOD) | | | | | | | | | |

**Table 13: Formulation C, 200 mg/mL stability: agitation, stressed at 40°C, accelerated at 25°C.**

| **Tests** | **Criteria** | **t0** | **agitatio n** | **1w, 40C** | **2w, 40C** | **1M, 40C** | **1w,25C** | **2w,25C** | **1M,25C** | **2M,25C** | **3M,25C** | **6M,25C** |
|---|---|---|---|---|---|---|---|---|---|---|---|---|
| icIEF - %Acidic | Report Result | 31.9 | NT | NT | NT | 45.7 | NT | NT | NT | NT | 39.1 | NT |
| icIEF - %Main | Report Result | 54.6 | NT | NT | NT | 39.4 | NT | NT | NT | NT | 44.5 | NT |
| icIEF - %Basic | Report Result | 13.5 | NT | NT | NT | 15.0 | NT | NT | NT | NT | 16.4 | NT |
| NR CE-SDS - %IgG | Report Result | NT | NT | NT | NT | 97.5 | NT | NT | NT | NT | 98.4 | NT |
| NR CE-SDS - %Highest Impurity | Report Result | NT | NT | NT | NT | 0.8 | NT | NT | NT | NT | 0.6 | NT |
| SEC%Main | ≥95% | 99.1 | 98.8 | 98.1 | 97.8 | 97.0 | 98.7 | 98.8 | 98.6 | 98.3 | 98.2 | 97.8 |
| SEC%HMW | Report Result | 0.9 | 1.2 | 1.8 | 2.1 | 2.9 | 1.3 | 1.2 | 1.5 | 1.7 | 1.8 | 2.2 |
| SEC%LMW | Report Result | ND | ND | 0.1 | 0.0 | 0.1 | ND | ND | ND | 0.0 | 0.0 | 0.1 |

| SVP analysis | | | | | | | | | | | | |
|---|---|---|---|---|---|---|---|---|---|---|---|---|
| ≥2to<10 µm | Report Result | 25 | 0 | 4980 | 7410 | 4005 | 4026 | 3760 | 6067 | 4565 | 12795 | NT |
| ≥10 µm | Report Result | 1 | 1 | 120 | 260 | 60 | 67 | 160 | 180 | 145 | 210 | NT |
| ≥25 µm | Report Result | 99 | 99 | 40 | 30 | 0 | 40 | 40 | 20 | 15 | 10 | NT |

| | | | | | | | | | | | | |
|---|---|---|---|---|---|---|---|---|---|---|---|---|
| NT = Not tested, ND = Not detectable (<LOD) | | | | | | | | | | | | |

**Table 14: Formulation C, 200 mg/mL stability, frozen at -20°C or -70°C.**

| **Tests** | **Criteria** | **t0** | **3M,-20C** | **3M,-70C** | **6M,-70C** | **9M,-70C** | **12M,-70C** |
|---|---|---|---|---|---|---|---|
| icIEF - %Acidic | Report Result | 31.9 | NT | NT | NT | 35.7 | 34.5 |
| icIEF - %Main | Report Result | 54.6 | NT | NT | NT | 47.2 | 48.6 |
| icIEF - %Basic | Report Result | 13.5 | NT | NT | NT | 17.1 | 16.9 |
| NR CE-SDS - %IgG | Report Result | NT | NT | NT | NT | 98.9 | 98.9 |
| NR CE-SDS - %Highest Impurity | Report Result | NT | NT | NT | NT | 0.6 | 0.6 |
| SEC%Main | ≥95% | 99.1 | 98.8 | 98.9 | NT | 99.0 | 99.0 |
| SEC%HMW | Report Result | 0.9 | 1.2 | 1.1 | NT | 1.0 | 1.0 |
| SEC%LMW | Report Result | ND | ND | ND | NT | ND | ND |

| SVP analysis | | | | | | | |
|---|---|---|---|---|---|---|---|
| ≥2to<10 µm | Report Result | 25 | 36415 | 15600 | NT | 150695 | 88307 |
| ≥10 µm | Report Result | 1 | 1165 | 500 | NT | 3490 | 1026 |
| ≥25 µm | Report Result | 99 | 100 | 35 | NT | 210 | 32 |

| | | | | | | | |
|---|---|---|---|---|---|---|---|
| NT = Not tested, ND = Not detectable (<LOD) | | | | | | | |

Subsequent aggregation data were produced for Formulation A (100 mg/mL KPL-404 in 50 mM sodium acetate, 7% (w/v) sucrose, 0.01% (w/v) polysorbate 20, pH 5.4) KPL-404 up to 24 months at 2-8 °C. In a first lot, the main peak in the SEC was 98.3% at T=0 and 97.6% at T=12 months and T=24 months, indicating a ΔSEC (main peak) of -0.71%, which was unchanged from 12 to 24 months. The high molecular weight (HMW) peak for the SEC, which represents aggregates of the antibody, was 1.3% at T=0, 1.6% at T=12 months, and 1.8% at T=24 months, indicating very little increase in aggregation over time. In a second lot, the main peak in the SEC was 98.6% at T=0 and 97.8% at T=12 months, indicating a ΔSEC (main peak) of -0.81%. Data were not produced to 24 months. The high molecular weight (HMW) peak for the SEC in the second lot was 0.9% at T=0, 1.4% at T=12 months, and 1.5% at T=18 months, also indicating very little increase in aggregation over time.

Aggregation data were also produced for Formulation C (200 mg/mL KPL-404 in 50 mM sodium acetate, 100 mM L-Arginine, 100 mM L-Glutamate, 0.02% polysorbate 20, pH 5.4) KPL-404 up to 12 months at 2-8 °C. In a first lot, the protein exhibited an SEC main peak of 99.2% and 98.9% at T=0 and T=12 months, respectively, indicating a ΔSEC (main peak) of -0.3%. The HMW peak was 0.60% at T=0 and 0.70% at T=12 months, indicating a slight increase in aggregation over time. In a second lot, the protein exhibited an SEC main peak of 98.7% and 98.1% at T=0 and T=9 months, respectively, indicating a ΔSEC (main peak) of -0.61%. The HMW peak was 0.40% at T=0 and 0.70% at T=9 months, indicating a slight increase in aggregation over time.

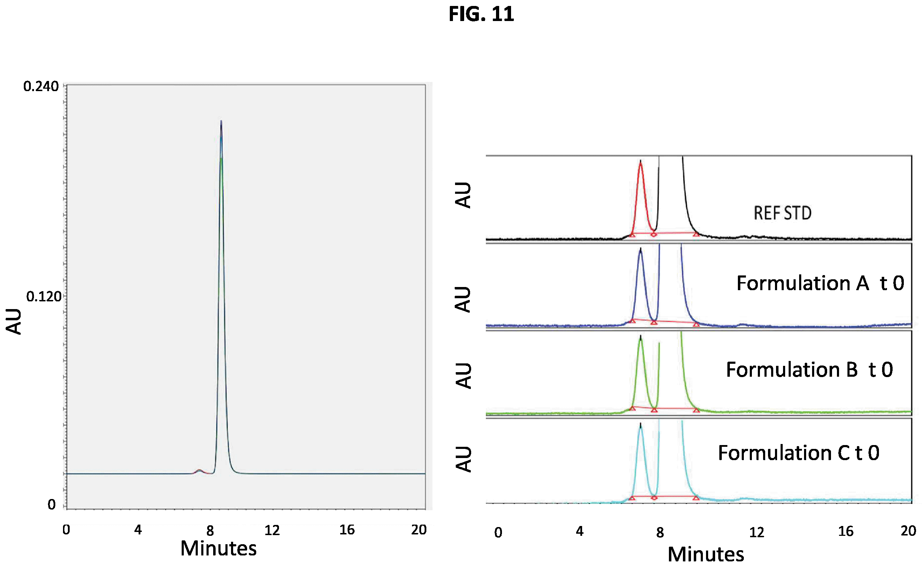

Aggregation by SEC is a major degradation pathway. **FIG. 1** shows a set of graphs showing SEC results presented graphically for %Main, %HMW and %LMW species of KPL-404 for Formulations A, 1 and 2. Chromatograms of initial samples are shown in **FIG. 11****,** showing initially all three formulations are essentially the same as the reference standard.

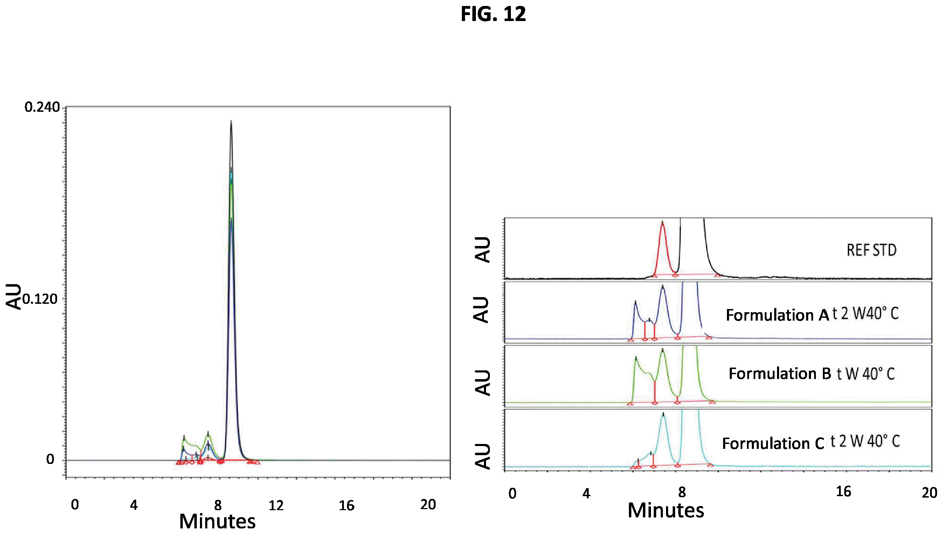

Following thermal stress at 40°C for 2 weeks, **FIG. 12****,** aggregation was observed for all three formulations, with species larger than a dimer formed for Formulations A and B. Formulation C minimized formation of higher-ordered species in comparison, consistent with the Arg/Glu excipients enabling higher protein concentrations of KPL-404 relative to sucrose and sorbitol.

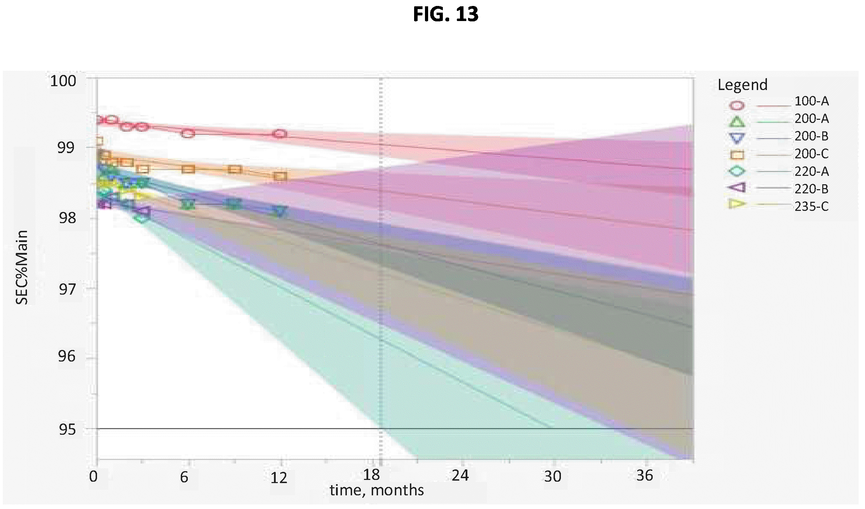

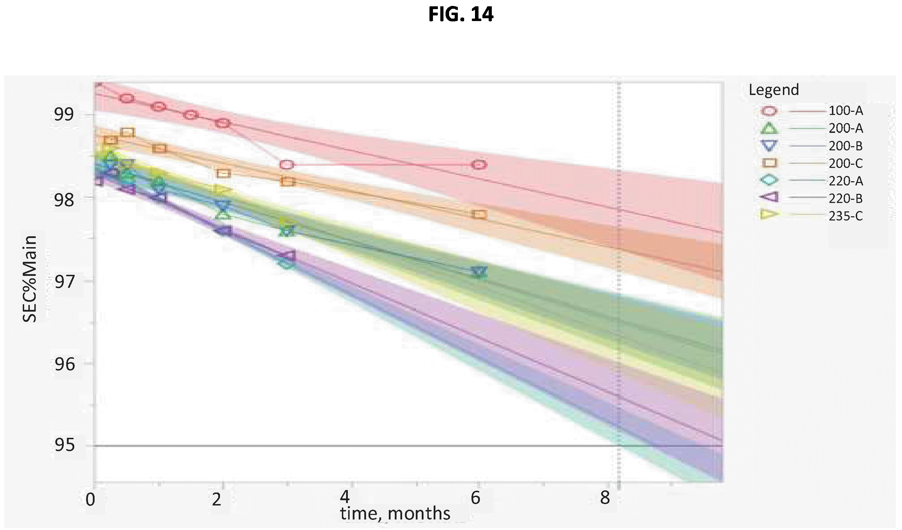

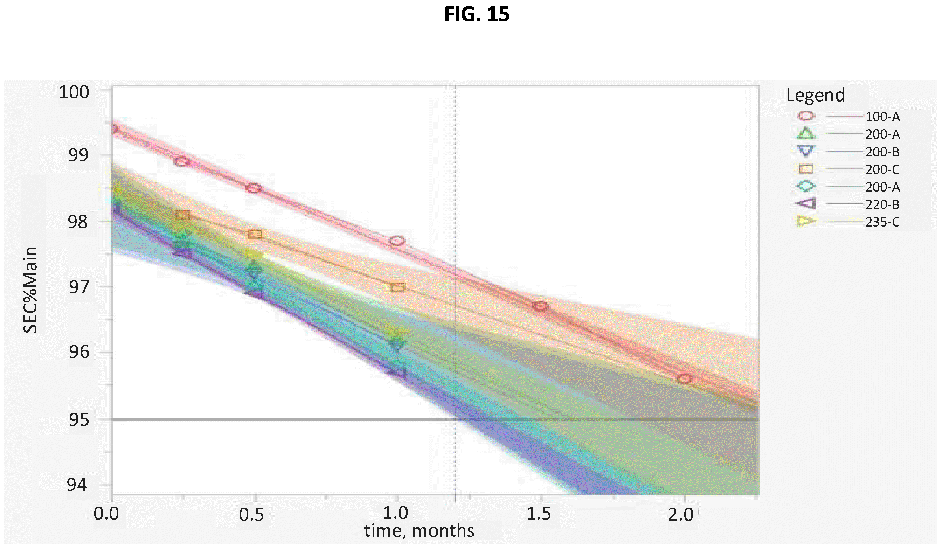

A statistical analysis was performed for SEC results from Formulations held at the recommended storage condition, 5°C **(****FIG. 13****),** at accelerated storage condition, 25°C **(****FIG. 14****),** at the stressed storage condition, 40°C **(****FIG. 15****),** and at frozen storage condition, -70°C **(F6).**

The statistical analysis assessed the Null Hypothesis (zero slope) with a one-sided 95% confidence interval; statistical significance was set at p-value < 0.05 for the slope being statistically non-zero, **Table 15.**

KPL-404 was stable at 5°C in the current formulation at 100 mg/mL, showing slow rate of change over 12 months with a slope that projects a ~1% change after 60 months. Of the three formulations at 200 mg/mL, Formulation C was more stable than both Formulations A and B; the slope for Formulation C predicts a ~1% change after ~36 months, whereas A and B project twice the rate of change as Formulation C. There is a sharp increase in loss of main through aggregation for all three formulations at 220 mg/mL for A and B, and 235 mg/mL for C.

Trends observed at 5°C were recapitulated in the accelerated stress condition at 25°C, with change of %Main occurring at a similar rate for Formulation A at 100 mg/mL and Formulation C at 200 mg/mL. The stability of KPL-404 in Formulation C at 235 mg/mL is consistently more stable than Formulations A and B at 220 mg/mL, whereas at 5°C they were essentially equivalent.

Trends observed at 40°C mirrored trends at 25°C and 5°C, with change of %Main occurring at a similar rate for Formulation A at 100 mg/mL and Formulation C at 200 mg/mL. Note that only Formulation A at 100 mg/mL had sufficient data for meaningful statistical analysis; analysis of higher concentration data sets are included in **Table 15** for qualitative comparison, which shows Formulation C was superior to Formulations A and B at reducing aggregation when held at the stressed storage condition 40°C. The results also show that even at elevated concentrations studied here, KPL-404 is a generally stable mAb against aggregation.

KPL-404 was stable at -70°C, with change of %Main occurring at a similar rate for Formulation A at 100 mg/mL and for Formulations A, B, and C at 200 mg/mL. Note that only Formulation A at 100 mg/mL had sufficient data for meaningful statistical analysis; analysis of higher concentration data sets are included in **Table 15** for qualitative comparison. The data predict no statistically meaningful change in %Main purity while held frozen, through 12 months of data.

**Table 15: Summary of P-values for Slope of SEC Results as a Function of Time.**

| **Temperature** | **Formulation** | **Concentration** | **Slope** | **P-value (slope)** |
|---|---|---|---|---|
| 5C | A | 100 mg/mL | -0.017 | 0.0809 |
| | | 200 mg/mL | -0.058 | <.0001 |
| | | 220 mg/mL | -0.114 | 0.0072 |
| | B | 200 mg/mL | -0.058 | <.0001 |
| | | 220 mg/mL | -0.034 | 0.3896 |
| | C | 200 mg/mL | -0.028 | 0.002 |
| | | 235 mg/mL | -0.071 | 0.0834 |
| 25C | A | 100 mg/mL | -0.171 | <.0001 |
| | | 200 mg/mL | -0.235 | <.0001 |
| | | 220 mg/mL | -0.383 | <.0001 |
| | B | 200 mg/mL | -0.23 | <.0001 |
| | | 220 mg/mL | -0.329 | <.0001 |
| | C | 200 mg/mL | -0.169 | <.0001 |
| | | 235 mg/mL | -0.275 | <.0001 |
| 40C | A | 100 mg/mL | -1.861 | <.0001 |
| | | 200 mg/mL | -2.029 | <.0001 ¹ |
| | | 220 mg/mL | -2.549 | <.0001 ¹ |
| | B | 200 mg/mL | -2.029 | <.0001 ¹ |
| | | 220 mg/mL | -2.48 | <.0001 ¹ |
| | C | 200 mg/mL | -1.486 | <.0001 ¹ |
| | | 235 mg/mL | -2.171 | <.0001 ¹ |
| -70C | A | 100 mg/mL | -0.0099 | 0.0656 |
| | | 200 mg/mL | 0.0119 | 0.1185¹ |
| | B | 200 mg/mL | 0.0024 | 0.7283¹ |
| | C | 200 mg/mL | 0.0119 | 0.1185¹ |

| | | | | |
|---|---|---|---|---|
| ¹ P-value is not meaningful with fewer than 5 data points, but is included for qualitative comparison | | | | |

In summary, KPL-404 is stable in all three formulations, but Formulation C is superior to both Formulation A and B in supporting concentrations above 200 mg/mL.

Studies were performed in three candidate formulations. KPL-404 was formulated at 200 mg/mL in each of the three candidate formulations by UF/DF and set on stability in a vial presentation.

KPL-404 was highly concentrated by UF/DF until the flux went to zero. The concentrations that were achieved were 272 and 274 mg/mL in Formulation A and B, respectively, and 291 mg/mL in Formulation C.

Samples of each of these were characterized externally using biophysical techniques available at Malvern Panalytical and at FDB.

These studies show that KPL-404 is well described by reversible monomer-dimer equilibrium below ~130 mg/mL.

At elevated concentrations from ~130 to 200 mg/mL, higher ordered reversible oligomers were formed in all three Formulations to varying degrees.

Viscosity and Instron testing were performed as a function of protein concentration to assess syringeability. Results showed Formulation C is more effective at reducing viscosity, and can support higher concentrations as compared to Formulations A and B.

Formulation C exhibits superior properties, either in a standard Yposmate autoinjector up to 235 mg/mL, or up to 270 mg/mL in the Ypsomate Pro.

All three formulations were placed on stability at 200 mg/mL, which is the upper concentration limit for Formulation A based on injectability data. Short-term (3 months) stability was also assessed at 220 mg/mL in Formulation A and B, and at 235 mg/mL in Formulation C.

Formulation C at 200 mg/mL was approximately as stable as Formulation A at 100 mg/mL.

Based on all results collected, Formulation C was identified as a superior formulation, relative to Formulations A and B, for KPL-404. An upper concentration limit of 250 mg/mL can be supported for drug substance final UF/DF.

### Example 2

### A 26-Week Repeat-Dose Toxicity and Toxicokinetic Study of KPL-404 Administered Once Weekly via Intravenous or Subcutaneous Injection to Cynomolgus Monkeys with an 8-Week Recovery Period

The objectives of this study were to evaluate the toxicity of KPL-404 prepared in Formulation C (200 mg/mL KPL-404, 50 mM Acetate, 100 mM L-Arginine, 100 mM L-Glutamate, 0.02% polysorbate 20, pH 5.4, when administered by intravenous or subcutaneous injection once weekly over a 26-week period to cynomolgus monkeys and to evaluate the potential reversibility or delayed occurrence of any effects over an 8-week recovery period. In addition, the toxicokinetic characteristics of the test article were determined. KPL-404 was administered to 5 male and 5 female cynomolgus monkey per group via subcutaneous (SC) injection or intravenous bolus (IV) injection at doses of 30 or 97 mg/kg, once weekly for 26 weeks, according to the study design in In-text Table 1. The control article, Formulation C without KPL-404, was given by SC injection at the same dosing frequency.

**Table 16: Experimental Design**

| **Group** | **Test Material** | **Route*** | **Dose Level (mg/kg)** | **Dose Conc. (mg/mL)** | **Dose Volume (mL/kg)** | **Terminal** | | **Recovery** | |
|---|---|---|---|---|---|---|---|---|---|
| | | | | | | **Male** | **Female** | **Male** | **Female** |
| 1 | Control | SC | 0 | 0 | 0.5 | 3 | 3 | 2 | 2 |
| 2 | KPL-404 | SC | 30 | 60 | 0.5 | 3 | 3 | 2 | 2 |
| 3 | KPL-404 | SC | 97 | 194 | 0.5 | 3 | 3 | 2 | 2 |
| 4 | KPL-404 | IV | 30 | 60 | 0.5 | 3 | 3 | 2 | 2 |
| 5 | KPL-404 | IV | 97 | 194 | 0.5 | 3 | 3 | 2 | 2 |

| | | | | | | | | | |
|---|---|---|---|---|---|---|---|---|---|
| *SC = Subcutaneous; IV = Intravenous | | | | | | | | | |

### Toxicology Results

No unexpected adverse effects were observed in cynomolgus monkeys treated with placebo, or with either the low 30 mg/kg or high doses97 mg/kg dose levels of KPL-404 in Formulation C; all effects observed were attributable to the pharmacological action of KPL-404.

### CONCLUSION

KPL-404 is stable in all three formulations, but Formulation C is superior to both Formulation A and B in providing stable formulations of KPL-404, and Formulation C was further demonstrated to be safe in non-human primate toxicity studies.

### Other Embodiments

While specific aspects of the invention have been described and illustrated, such aspects should be considered illustrative of the invention only and not as limiting the invention as construed in accordance with the accompanying claims. All publications and patent applications cited in this specification are herein incorporated by reference in their entirety for all purposes as if each individual publication or patent application were specifically and individually indicated to be incorporated by reference for all purposes. Although the foregoing invention has been described in some detail by way of illustration and example for purposes of clarity of understanding, it will be readily apparent to one of ordinary skill in the art in light of the teachings of this invention that certain changes and modifications can be made thereto without departing from the spirit or scope of the appended claims.

The following pages 41 to 46 contain further embodiments.
1. A pharmaceutical composition comprising a humanized anti-CD40 antibody or antigen-binding fragment thereof comprising a heavy chain variable region comprising the amino acid sequence set forth in SEQ ID NO: 9, and a light chain variable region comprising the amino acid sequence set forth in SEQ ID NO: 10, wherein the pharmaceutical composition comprises polysorbate 20, acetate, and a polar excipient.
2. The pharmaceutical composition of item 1, wherein the pharmaceutical composition comprises from about 5 mM to about 100 mM acetate.
3. The pharmaceutical composition of item 2, wherein the pharmaceutical composition comprises about 50 mM acetate.
4. The pharmaceutical composition of any one of items 1-3, wherein the pharmaceutical composition comprises from about 0.001% to about 0.1% polysorbate 20.
5. The pharmaceutical composition of item 4, wherein the pharmaceutical composition comprises about 0.01% polysorbate 20 or about 0.02% polysorbate 20.
6. The pharmaceutical composition of any one of items 1-5, wherein the pharmaceutical composition comprises about 50 mM acetate and about 0.02% polysorbate 20.
7. The pharmaceutical composition of any one of items 1-6, wherein the pharmaceutical composition has a pH of about 5.0 to about 6.0.
8. The pharmaceutical composition of item 7, wherein the pharmaceutical composition has a pH of about 5.4.
9. The pharmaceutical composition of item 1, wherein the polar excipient comprises arginine and glutamate.
10. The pharmaceutical composition of item 9, wherein the pharmaceutical composition comprises from about 5 mM to about 100 mM acetate.
11. The pharmaceutical composition of item 10, wherein the pharmaceutical composition comprises about 50 mM acetate.
12. The pharmaceutical composition of any one of items 9-11, wherein the pharmaceutical composition comprises from about 1 mM to about 200 mM arginine.
13. The pharmaceutical composition of item 12, wherein the pharmaceutical composition comprises about 100 mM arginine.
14. The pharmaceutical composition of any one of items 9-13, wherein the pharmaceutical composition comprises from about 1 mM to about 200 mM glutamate.
15. The pharmaceutical composition of item 14, wherein the pharmaceutical composition comprises about 100 mM glutamate.
16. The pharmaceutical composition of any one of items 9-15, wherein the pharmaceutical composition comprises from about 0.001% to about 0.1% polysorbate 20.
17. The pharmaceutical composition of item 16, wherein the pharmaceutical composition comprises about 0.01% polysorbate 20 or about 0.02% polysorbate 20.
18. The pharmaceutical composition of any one of items 9-17, wherein the pharmaceutical composition comprises about 50 mM acetate, about 100 mM arginine, about 100 mM glutamate, and about 0.02% polysorbate 20.
19. The pharmaceutical composition of any one of items 9-18, wherein the pharmaceutical composition has a pH of about 5.0 to about 6.0.
20. The pharmaceutical composition of item 19, wherein the pharmaceutical composition has a pH of about 5.4.
21. The pharmaceutical composition of item 1, wherein the polar excipient comprises sucrose.
22. The pharmaceutical composition of item 21, wherein the pharmaceutical composition comprises from about 5 mM to about 100 mM acetate.
23. The pharmaceutical composition of item 22, wherein the pharmaceutical composition comprises about 50 mM acetate.
24. The pharmaceutical composition of any one of items 21-23, wherein the pharmaceutical composition comprises from about 1% (w/v) to about 10% (w/v) sucrose.
25. The pharmaceutical composition of item 24, wherein the pharmaceutical composition comprises about 7% (w/v) sucrose.
26. The pharmaceutical composition of any one of items 21-25, wherein the pharmaceutical composition comprises from about 0.001% to about 0.1% polysorbate 20.
27. The pharmaceutical composition of item 26, wherein the pharmaceutical composition comprises about 0.01% polysorbate 20 or about 0.02% polysorbate 20.
28. The pharmaceutical composition of any one of items 21-27, wherein the pharmaceutical composition comprises about 50 mM acetate, about 7% (w/v) sucrose, and about 0.01% polysorbate 20 or 0.02% polysorbate 20.
29. The pharmaceutical composition of any one of items 21-28, wherein the pharmaceutical composition has a pH of about 5.0 to about 6.0.
30. The pharmaceutical composition of item 29, wherein the pharmaceutical composition has a pH of about 5.4.
31. The pharmaceutical composition of item 1, wherein the polar excipient comprises sorbitol.
32. The pharmaceutical composition of item 31, further comprising sodium chloride.
33. The pharmaceutical composition of item 32, wherein the pharmaceutical composition comprises from about 1 mM to about 100 mM sodium chloride.
34. The pharmaceutical composition of item 33, wherein the pharmaceutical composition comprises about 70 mM sodium chloride.
35. The pharmaceutical composition of any one of items 31-34, wherein the pharmaceutical composition comprises from about 5 mM to about 100 mM acetate.
36. The pharmaceutical composition of item 35, wherein the pharmaceutical composition comprises about 50 mM acetate.
37. The pharmaceutical composition of any one of items 31-36, wherein the pharmaceutical composition comprises from about 0.1% (w/v) sorbitol to about 5% (w/v) sorbitol.
38. The pharmaceutical composition of item 37, wherein the pharmaceutical composition comprises about 2.5% (w/v) sorbitol.
39. The pharmaceutical composition of any one of items 31-38, wherein the pharmaceutical composition comprises from about 0.001% to about 0.1% polysorbate 20.
40. The pharmaceutical composition of item 39, wherein the pharmaceutical composition comprises about 0.01% polysorbate 20 or about 0.02% polysorbate 20.
41. The pharmaceutical composition of any one of items 32-40, wherein the pharmaceutical composition comprises about 50 mM acetate, about 2.5% (w/v) sorbitol, about 70 mM NaCl, and about 0.01% polysorbate 20 or 0.02% polysorbate 20.
42. The pharmaceutical composition of any one of items 31-41, wherein the pharmaceutical composition has a pH of about 5.0 to about 6.0.
43. The pharmaceutical composition of item 42, wherein the pharmaceutical composition has a pH of about 5.4.
44. The pharmaceutical composition of any one of items 1-43, wherein the antibody or antigen-binding fragment thereof is at a concentration of from about 50 mg/mL to about 300 mg/mL.
45. The pharmaceutical composition of item 44, wherein the antibody or antigen-binding fragment thereof is at a concentration of about 200 mg/mL.
46. The pharmaceutical composition of any one of items 1-45, wherein the composition is formulated in a volume of about 0.1 mL to about 5.0 mL.
47. The pharmaceutical composition of item 46, wherein the composition is formulated in a volume of about 2.0 mL.
48. The pharmaceutical composition of any one of items 1-47, wherein the humanized anti-CD40 antibody or antigen-binding fragment thereof is KPL-404.
49. The pharmaceutical composition of any one of items 1-48, wherein at least 95% of the humanized anti-CD40 antibody or antigen-binding fragment thereof is present in a monomer form.
50. The pharmaceutical composition of any one of items 1-49, wherein less than 5% of the humanized anti-CD40 antibody or antigen-binding fragment is present as a high molecular weight (HMW) species.
51. The pharmaceutical composition of item 49 or 50, wherein at least 98% of the humanized anti-CD40 antibody or antigen-binding fragment thereof is present in a monomer form.
52. The pharmaceutical composition of item 51, wherein less than 2% of the humanized anti-CD40 antibody or antigen-binding fragment is present as an HMW species.
53. The pharmaceutical composition of item 49 or 50, wherein an amount of the monomeric form of the humanized anti-CD40 antibody or antigen-binding fragment in the composition decreases to no less than 95% following storage at 2-8 °C for 12 months.
54. The pharmaceutical composition of item 53, wherein an amount of the HMW species of the humanized anti-CD40 antibody or antigen-binding fragment in the composition increases to no more than 5% following storage at 2-8 ° C for 12 months.
55. The pharmaceutical composition of any one of items 51-54, wherein the amount of the monomer form of the humanized anti-CD40 antibody or antigen-binding fragment in the composition decreases to no less than 98% following storage at 2-8 °C for 12 months.
56. The pharmaceutical composition of item 55, wherein the amount of theHMW species of the humanized anti-CD40 antibody or antigen-binding fragment in the composition increases to no more than 2% following storage at 2-8 ° C for 12 months.
57. A kit comprising the pharmaceutical composition of any one of items 1-56 and instructions for use thereof.
58. Use of the pharmaceutical composition of any one of items 1-56 in the manufacture of a medicament for suppressing the immune system in a human subject.
59. The pharmaceutical composition of any one of items 1-56 for use in suppressing the immune system in a human subject.
60. A method of suppressing the immune system in a human subject comprising administering the pharmaceutical composition of any one of items 1-56 to the subject.
61. The method of item 60, wherein the subject is a human.

## Claims

1. A pharmaceutical composition comprising a humanized anti-CD40 antibody or antigen-binding fragment thereof comprising a heavy chain variable region comprising the amino acid sequence set forth in SEQ ID NO: 9, and a light chain variable region comprising the amino acid sequence set forth in SEQ ID NO: 10, wherein the pharmaceutical composition comprises polysorbate 20, acetate, and a polar excipient.

2. The pharmaceutical composition of claim 1, wherein the pharmaceutical composition comprises from about 5 mM to about 100 mM acetate, wherein, optionally, the pharmaceutical composition comprises about 50 mM acetate.

3. The pharmaceutical composition of claim 1 or 2, wherein the pharmaceutical composition comprises from about 0.001% to about 0.1% polysorbate 20, wherein, optionally, the pharmaceutical composition comprises about 0.02% or about 0.01% polysorbate 20.

4. The pharmaceutical composition of any one of claims 1-3, wherein the pharmaceutical composition has a pH of about 5.0 to about 6.0, wherein, optionally, the pharmaceutical composition has a pH of about 5.4.

5. The pharmaceutical composition of any one of claims 1 to 4, wherein the polar excipient comprises arginine and glutamate, wherein, optionally, the pharmaceutical composition comprises from about 1 mM to about 200 mM arginine, such as about 100 mM arginine or from about 1 mM to about 200 mM glutamate, such as about 100 mM glutamate.

6. The pharmaceutical composition of claim 5, wherein the pharmaceutical composition comprises about 50 mM acetate, about 100 mM arginine, about 100 mM glutamate, and about 0.02% polysorbate 20.

7. The pharmaceutical composition of any one of claims 1-4, wherein the polar excipient comprises sucrose, wherein, optionally, the pharmaceutical composition comprises from about 1% (w/v) to about 10% (w/v) sucrose, such as about 7% (w/v) sucrose.

8. The pharmaceutical composition of claim 7, wherein the pharmaceutical composition comprises about 50 mM acetate, about 7% (w/v) sucrose, and about 0.02% or about 0.01% polysorbate 20.

9. The pharmaceutical composition of any one of claims 1-4, wherein the polar excipient comprises sorbitol, wherein, optionally, the pharmaceutical composition comprises from about 0.1% (w/v) sorbitol to about 5% (w/v) sorbitol, such as about 2.5% (w/v) sorbitol.

10. The pharmaceutical composition of claim 9, further comprising sodium chloride, wherein, optionally, the pharmaceutical composition comprises from about 1 mM to about 100 mM sodium chloride, such as about 70 mM sodium chloride.

11. The pharmaceutical composition of any one of claims 10, wherein the pharmaceutical composition comprises about 50 mM acetate, about 2.5% (w/v) sorbitol, about 70 mM NaCl, and about 0.02% or about 0.01% polysorbate 20.

12. The pharmaceutical composition of any one of claims 1-11, wherein the antibody or antigen-binding fragment thereof is at a concentration of from about 50 mg/mL to about 300 mg/mL, wherein optionally, the antibody or antigen-binding fragment thereof is at a concentration of about 200 mg/mL

13. The pharmaceutical composition of any one of claims 1-12, wherein:
a) the humanized anti-CD40 antibody or antigen-binding fragment thereof is KPL-404;
b) at least 95% of the humanized anti-CD40 antibody or antigen-binding fragment thereof is present in a monomer form;
c) less than 5% or less than 2% of the humanized anti-CD40 antibody or antigen-binding fragment is present as a high molecular weight (HMW) species; and/or
d) wherein an amount of the HMW species of the humanized anti-CD40 antibody or antigen-binding fragment in the composition increases to no more than 5% or 2% following storage at 2-8 ° C for 12 months.

14. The pharmaceutical composition of any one of claims 1-13 for use as a medicament.

15. The pharmaceutical composition of any one of claims 1-13 for use in suppressing the immune system in a human subject.
